# EUROPEAN PATENT SPECIFICATION

(11) **EP 2 000 855 B1**
(45) Date of publication and mention of the grant of the patent: **03.08.2011**
(21) Application number: 08157717.3
(22) Date of filing: 06.06.2008
(51) Int. Cl.: G03G 5/04, G03G 5/06, G03G 5/147

(54) **Image bearing member, method of manufacturing the same, image formation method, image forming apparatus and process cartridge**
Bildträgerelement, Herstellungsverfahren dafür, Bilderzeugungsverfahren, Bilderzeugungsvorrichtung und Prozesskartusche
Élément supportant une image, son procédé de fabrication, procédé et appareil de formation d'images et cartouche de traitement

(30) Priority: 07.06.2007 JP 2007151766; 12.05.2008 JP 2008125017
(43) Date of publication of application: 10.12.2008
(73) Proprietor: Ricoh Company, Ltd., Tokyo 143-8555 (JP)
(72) Inventor: Nagai, Kazukiyo, Tokyo 143-8555 (JP); Horiuchi, Tamotsu, Tokyo 143-8555 (JP); Suzuki, Tetsuro, Tokyo 143-8555 (JP); Ikuno, Hiroshi, Tokyo 143-8555 (JP); Li, Hongguo, Tokyo 143-8555 (JP); Mori, Nobuya, Tokyo 143-8555 (JP)
(74) Representative: Barz, Peter

(56) References cited:
- EP-A- 1 600 822
- US-A1- 2006 051 688

## Description

### BACKGROUND OF THE INVENTION

### Field of the Invention

The present invention relates to an image bearing member and an image formation method, an image forming apparatus and a process cartridge using the same.

### Discussion of the Background

In recent years, an organic photoconductor (OPC) has superseded an inorganic photoconductor in a photocopier, a facsimile machine, a laser printer and their multi-functional device as an image bearing member in light of performance and advantages. For example, such an organic photoconductor has the following advantages over an inorganic photoconductor: (1) a wide light absorption wavelength range and a large light absorption amount with regard to optical characteristics; (2) high sensitivity and stable chargeability with regard to electric characteristics; (3) wide selection range of material; (4) easiness of manufacturing; (5) low cost; and (6) non-toxic. On the other hand, along with the advance of size reduction of an image forming apparatus, the image bearing member therein has been also reduced in size. Furthermore, due to movement toward high speed performance and maintenance-free, an image bearing member having a high durability has been strongly demanded. In light of this point, an organic photoconductor is generally soft because the main components of the charge transport layer of such an organic photoconductor are a low molecular weight charge transport material and an inactive polymer. Therefore, when such an organic photoconductor is repetitively used in the electrophotographic process, the organic photoconductor has a disadvantage of being easily abraded under the mechanical stress caused by a development system or a cleaning system. In addition, to meet the demand of producing quality images, the size of toner particles decreases, which requires improvement of cleaning property. Thus, the hardness of rubber of a cleaning blade and the contact pressure thereof to an image bearing member increases. This results in another factor of accelerating the abrasion of the surface of an image bearing member. Such abrasion of an image bearing member invites deterioration of electric characteristics such as sensitivity and chargeability and causes reduction of image density, background fouling, etc., which leads to production of abnormal images. When such abrasion occurs locally and damages the surface of an image bearing member, obtained images have streak fouling. Therefore, various kinds of attempts and studies have been made to improve the anti-abrasion property of an organic photoconductor.

For example, Japanese patent No. (hereinafter referred to as JP) 3262488 describes an image bearing member having a cross linking surface layer formed by curing a radical curing monomer having multiple function groups. There are notable things about an image bearing member using such a monomer having multiple function groups such that high anti-abrasion characteristics are obtained because dense three dimensional network structure is formed, cured layer can be formed instantly upon application of light, heat and radiation, and the curing material does not require a catalyst for an acid or a base in the curing reaction, thereby avoiding an adverse impact thereof on electric characteristics. Furthermore, JP 3194392, unexamined published Japanese patent application No. (hereinafter referred to as JOP) 2000-66425 and US 20060051688 A1 describe a technology in which a charge transport material having a radical polymerizable function group is used in combination and thus the charge transport structure is fixed in the cross linking structure, resulting in improvement on the electric characteristics of the cross linking surface layer. According to these technologies, it is expected to manufacture an image bearing member having a good combination of anti-abrasion property and charge transportability.

However, it is found that various kinds of characteristics are affected according to the kind of the charge transport material having polymerizable function groups. Especially, in the case in which a cross linking surface layer is formed by optical curing, irradiation of light is greatly influential. In general, ultraviolet is used as a light source for optical curing. Part of the charge transport material is dissembled by the ultraviolet, during which various kinds of dissembled materials are produced determined depending on the structure of the charge transport material. Part of these dissembled materials remains in the cross-linking surface layer, which causes deterioration of the characteristics. Since the kinds of dissembled materials are determined depending on the structure of a charge transport material, the resultant characteristics are greatly dependent on the kind of the charge transport material.

In addition, introduction of reaction groups into a charge transport material greatly affects the charge transportability and the mechanical characteristics of a cross-linking layer depending on the connection position and the structure. Therefore, it is difficult to obtain a good combination of the anti-abrasion property and the charge transportability when radical reactive compounds are arbitrarily selected.

By contrast, a cross linking surface layer formed of a cured material has a highly cross linked three dimensional network structure and thus obtain a high anti-abrasion property. Such a cured material originally contains polar groups in a large amount and has a large dielectric constant. This leads to problems of deterioration of electric characteristics such as decrease in the resistivity and/or sensitivity, and image deterioration such as density change, image flow and decrease in definition due to environmental change with regard to humidity, temperature, etc., and an acid gas produced by a charging device. The structure of a radical reactive compound is also influential in this case.

Furthermore, it is possible to elongate the life length of an image bearing member against abrasion by thickening the cross linking surface layer thereof. In this case, it is preferred to have an excellent photosensitivity by which the irradiation potential in an image forming apparatus can be sufficiently lowered. This makes it more difficult to have a good combination of the anti-abrasion property and the charge transportability.

To solve these drawbacks, it is desired to form a crosslinked surface layer that satisfies every kind of characteristics. Thus, it is important to select a radical reactive compound that forms such a crosslinked surface layer. However, a sufficiently satisfactory radical reactive compound has not been found and thus a crosslinked surface layer having a good combination of the anti-abrasion property and the charge transportability at a high level and an image bearing member formed of a crosslinked surface layer have not been obtained.

### SUMMARY OF THE INVENTION

Because of these reasons, the present inventors recognize that a need exists for an image bearing member having a good combination of the anti-abrasion property, the electric characteristics and the adaptability to environmental change.

Accordingly, an object of the present invention is to provide an image bearing member that has a good combination of the anti-abrasion property, the electric characteristics and the adaptability to environmental change. To be more specific, it is to provide an image bearing member that has a crosslinked surface layer formed by using a particular radical reactive compound, maintains high anti-abrasion property and the variation in the irradiation voltage in a small range even when the thickness of the crosslinked layer is increased, is stable against the environment humidity and temperature and an oxidized gas produced from a charging device, and hardly causes image densityvariation, image flow or definition decrease. Another object of the present invention is to provide a method of manufacturing the image bearing member and furthermore an image formation method, an image forming apparatus and a process cartridge by which quality images can be produced for an extended period of time with the highly stable and log life image bearing member mentioned above. Briefly these objects and other objects of the present invention as hereinafter described will become more readily apparent and can be attained, either individually or in combination thereof, by an image bearing member comprising an electroconductive substrate, a photosensitive layer located overlying the electroconductive substrate and a crosslinked surface layer comprising a charge transport curing compound and located overlying the photosensitive layer, wherein the charge transport curing compound includes a material including a structure unit represented by the following Chemical formula A: 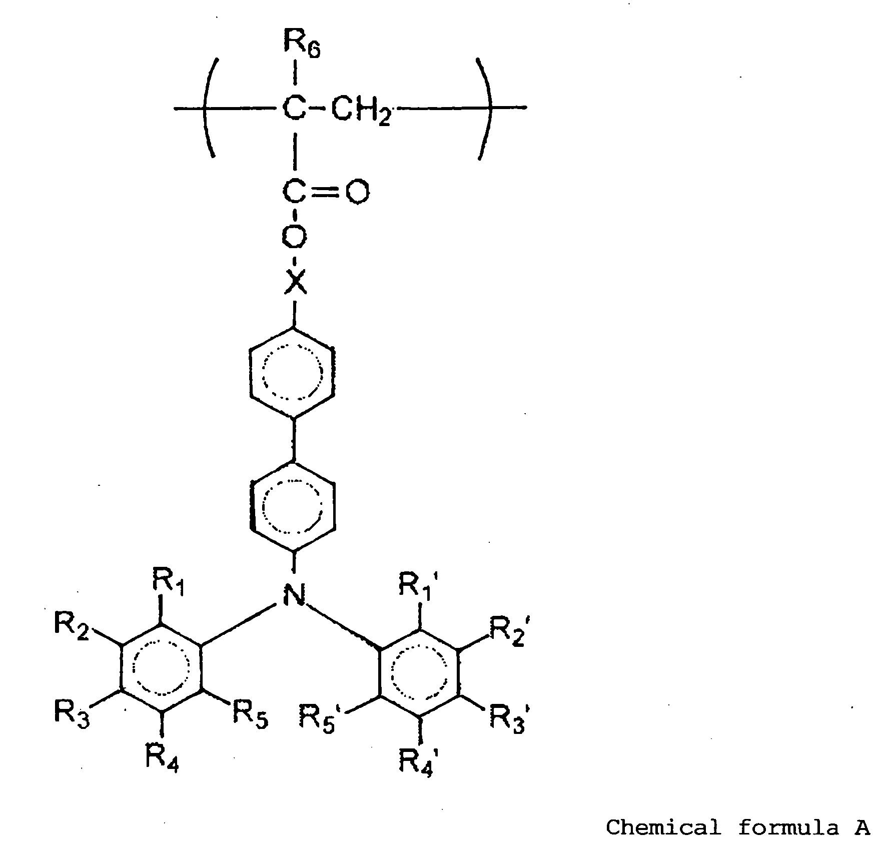 where R₁ to R₅ and R₁' to R₅' independently represent a hydrogen atom, an alkyl group having 1 to 4 carbon atoms or an alkoxy group having 1 to 4 carbon atoms, R₆ represents a hydrogen atom or a methyl group and X represents an alkylene group having a straight chain or a branched chain having 2 to 5 carbon atoms.

It is preferred that, in the image bearing member, X is an ethylene group and a phenyl group having R₁ to R₅ groups is different from a phenyl group having R₁' to R₅' groups.

It is still further preferred that, in the image bearing member, X is a straight chained alkylene group having 3 to 5 carbon atoms.

It is still further preferred that, in the image bearing member, the charge transport curing compound is formed by applying a liquid application including the compound represented by Chemical formula A and a monomer having at least 3 polymerizable function groups to the surface of the photosensitive layer followed by curing.

It is still further preferred that, in the image bearing member, the liquid application further includes a polymerization initiator.

It is still further preferred that, in the image bearing member, the cross linking surface layer is formed on the photosensitive layer and the photosensitive layer is uncured.

It is still further preferred that, in the image bearing member, the photosensitive layer has a structure in which a charge generation layer and a charge transport layer are sequentially accumulated on the electroconductive substrate.

As another aspect of the present invention, a method of manufacturing an image bearing member including coating a liquid application including a radical reactive compound represented by the following Chemical formula B to the surface of the photosensitive layer of the image bearing member mentioned above; and curing the liquid application to form the charge transport curing compound; 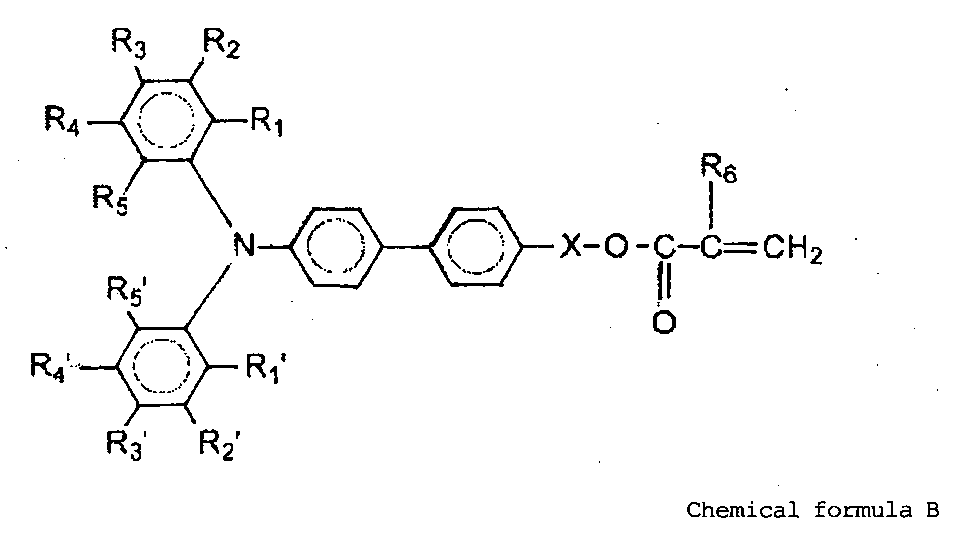 where R₁ to R₅ and R₁' to R₅' independently represent a hydrogen atom, an alkyl group having 1 to 4 carbon atoms or an alkoxy group having 1 to 4 carbon atoms, R₆ represents a hydrogen atom or a methyl group and X represents an alkylene group having 2 to 5 carbon atoms with a straight chain or a branched chain.

Is it preferred that, in the method of manufacturing an image bearing member, X in the chemical formula B is an ethylene group and a phenyl group having R₁ to R₅ groups is different from a phenyl group having R₁' to R₅' groups.

Is it still further preferred that, in the method of manufacturing an image bearing member, X is a straight chained alkylene group having 3 to 5 carbon atoms.

As another aspect of the present invention, an image formation method is provided including charging the image bearing member mentioned above, irradiating the image bearing member to form a latent electrostatic image on the image bearing member, developing the latent electrostatic image with a developing agent to form a developed image and transferring the developed image to a recording medium.

As another aspect of the present invention, an image forming apparatus is provided which includes the image bearing member mentioned above, a charging device configured to charge the image bearing member, an irradiation device configured to irradiate the image bearing member with light to form a latent electrostatic image on the image bearing member, a development device configured to develop the latent electrostatic image with a developing agent, and a transfer device configured to transfer the developed image to a recording medium.

As another aspect of the present invention, a process cartridge is provided which include the image bearing member mentioned above and at least one device selected from the group consisting of a charging device, an irradiation device, a development device, a transfer device, a cleaning device and a discharging device, wherein the image bearing member and the at least one device are integrally structured and the process cartridge is detachably attachable to an image forming apparatus.

As another aspect of the present invention, a radical reactive compound represented by chemical formula B illustrated above in the method of manufacturing a member mentioned above. When the number of carbon atoms in X is two, the phenyl group having R₁ to R₅ groups is different from the phenyl group having R₁' to R₅' groups.

These and other objects, features and advantages of the present invention will become apparent upon consideration of the following description of the preferred embodiments of the present invention taken in conjunction with the accompanying drawings.

### BRIEF DESCRIPTION OF THE DRAWINGS

Various other objects, features and attendant advantages of the present invention will be more fully appreciated as the same becomes better understood from the detailed description when considered in connection with the accompanying drawings in which like reference characters designate like corresponding parts throughout and wherein:
Fig. 1 is a diagram illustrating an example of the layer structure of the image bearing member of the present invention;
Fig. 2 is a diagram illustrating another example of the layer structure of the image bearing member of the present invention;
Fig. 3 is a schematic diagram illustrating an example of the image forming apparatus of the present invention;
Fig. 4 is a schematic diagram illustrating an example of the process cartridge of the present invention;
Fig. 5 is a diagram illustrating an IR (infra red) spectrum of the illustrated compound No. 1, which is described later;
Fig. 6 is a diagram illustrating an IR (infra red) spectrum of the illustrated compound No. 2, which is described later;
Fig. 7 is a diagram illustrating an IR (infra red) spectrum of the illustrated compound No. 8, which is described later;
Fig. 8 is a diagram illustrating an IR (infra red) spectrum of the illustrated compound No. 9, which is described later;
Fig. 9 is a diagram illustrating an IR (infra red) spectrum of the illustrated compound No. 10, which is described later;
Fig. 10 is a diagram illustrating an IR (infra red) spectrum of the illustrated compound No. 13, which is described later;
Fig. 11 is a diagram illustrating an IR (infra red) spectrum of the illustrated compound No. 14, which is described later;
Fig. 12 is a diagram illustrating an IR (infra red) spectrum of the illustrated compound No. 16, which is described later;
Fig. 13 is a diagram illustrating an IR (infra red) spectrum of the illustrated compound No. 26, which is described later;
Fig. 14 is a diagram illustrating an IR (infra red) spectrum of the illustrated compound No. 30, which is described later; and
Fig. 15 is a diagram illustrating an IR (infra red) spectrum of the illustrated compound No. 35, which is described later.

### DETAILED DESCRIPTION OF THE INVENTION

The present invention will be described below in detail with reference to several embodiments and accompanying drawings.

In the present invention, it is found that, by introducing a chemical compound having the following chemical formula A in the structure of the cured compound forming a cross linking surface layer, it is possible to provide an image bearing member which has an excellent anti-abrasion property, sufficient optical decay characteristics for the voltage at the development portion even when the layer thickness is increased, and a small variation in the optical decay even when the image bearing member is exposed to an oxidized gas such as ozone gas and nitrogen oxide gas (NOₓ gas). Thus, the provided image bearing member has a long life with less production of abnormal images caused by variation in image density, etc.

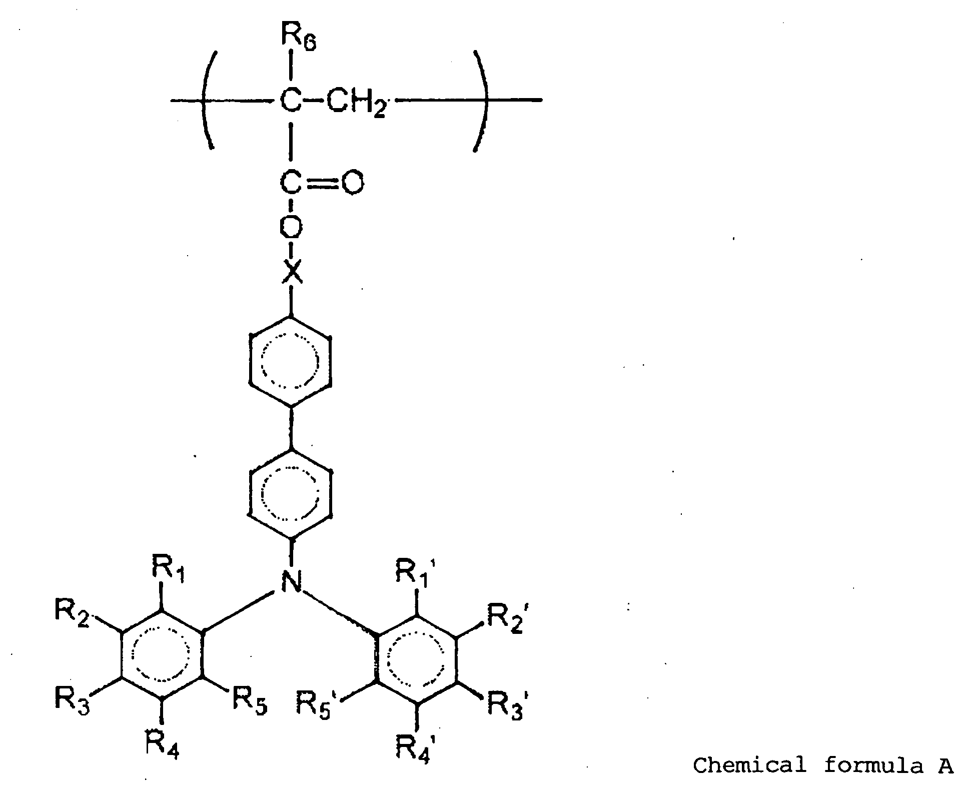

In Chemical formula A, R₁ to R₅ and R₁' to R₅' independently represent a hydrogen atom, an alkyl group having 1 to 4 carbon atoms or an alkoxy group having 1 to 4 carbon atoms, R₆ represents a hydrogen atom or a methyl group and X represents an alkylene group having a straight chain or a branched chain having 2 to 5 carbon atoms.

### Combination of Anti-abrasion Property and Charge Transport Property

It is known that a crosslinked layer formed of a cured (crosslinked) compound typically forms a dense three dimensional network structure, thereby having an excellent anti-abrasion property. To improve the anti-abrasion property, it is good to increase the amount of radical curing function groups in a cured composition material. For example, by using a monomer having a small acryl equivalent amount (the value obtained by dividing the monomer molecular weight with the number of its function groups) in the case of an acryl group, a dense three dimensional crosslinked structure is formed and a good anti-abrasion property is obtained JP 3262488 and unexamined published Japanese patent application No. (hereinafter referred to as JOP) 2006-227761}. In addition, it is effective to improve the anti-abrasion property using a monomer having multiple function groups, for example, a monomer having three or more function groups, furthermore, a monomer having six function groups. This is because the probability of increasing a molecular weight is high when at least one of the function groups is polymerized.

To form a layer having such a high cross linking density by curing, ultraviolet curing is preferred to thermal curing. Thus, it is desired to use a material which is transmissive and durable to ultraviolet irradiation. The reactive compound for use in the present invention is transmissive to ultraviolet. Therefore, this reactive compound is possibly relatively durable to ultraviolet irradiation in comparison with typically used compounds. The reason is not clear but it is inferred that, by an alkylene structure introduced between an acryloyloxy group and a charge transport function group, the thermal mobility of the reactive compound is heightened and thus the cross linking reaction during polymerization curing is promoted and the energy deactivation caused by thermal agitation when photoexcited prevents dissembling of compounds and changes the kinds of dissembled compounds so that a crosslinked layer which has relatively a small affect on the charge transport property is formed. In addition, it is also inferred that the selected charge transport group contains no aliphatic double bond structure but has a structure in which an acryloyloxy group is connected to the triphenyl amine structure, which is the center of charge transport, via a phenylene group and an alkylene group and thus, the structure corresponding to the charge transport group portion is not changed by severing of the double bond upon irradiation of ultraviolet, meaning that the structure maintains the original characteristics of the charge transport group.

### Environmental Stability

Next, the reason why the crosslinked surface layer for use in the present invention is durable against environmental change and an oxidized gas is described.

It is presumed that most of the materials dissembled upon irradiation of ultraviolet have a structure with a large polarity. Therefore, when the polar component in a layer increases, moisture, an oxidized gas produced from a charging device, etc., tend to enter inside the layer. Thereby, the resistance varies and the environment change is expected to increasingly affect the voltage. To the contrary, the radical reactive compound for use in the present invention is durable for ultraviolet irradiation, which prevents an increase of the polar composition in the layer. Therefore, the crosslinked surface layer is thought to be stable against environmental change.

### Cured Compound

Next, the composition component of the cured compound forming the crosslinked surface layer for use in the present invention is described.

In the present invention, the material having the structure unit represented by the chemical formula A illustrated above is included in the cured compound mentioned above. The reactive compound having the structure unit is cured by energy of, for example, light, heat or electron beam and introduced into the cured compound structure in the crosslinked surface layer. Upon this curing, the structure unit of Chemical formula A is fixed without being dissembled. This is confirmed by, for example, MS spectrum measurement by thermal cracking GC analysis or a characteristic absorption measurement by infrared spectroscopic analysis on the cured surface layer. The content of the structure unit of Chemical formula (A) is from 20 to 80 % by weight and preferably from 30 to 70 % by weight based on the total weight of the cured compound. When the content of this structure unit is excessively small, it tends to be difficult to obtain charge transport characteristics sufficient for an increase in layer thickness or reduce the occurrence of image deterioration. When the content of this structure unit is excessively large, the number of curing function groups tends to be small so that the mechanical strength such as anti-abrasion property and anti-damage property of the layer decreases.

A preferred example as the reactive compound is a compound represented by Chemical formula B.

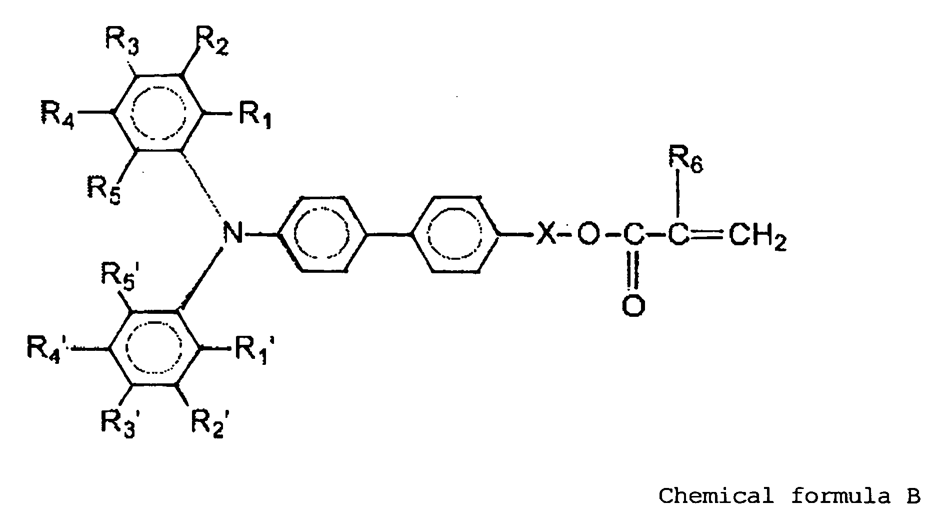

In the Chemical formula, R₁ to R₅ and R₁' to R₅' independently represent a hydrogen atom, an alkyl group having 1 to 4 carbon atoms or an alkoxy group having 1 to 4 carbon atoms, R₆ represents a hydrogen atom or a methyl group and X represents an alkylene group having a straight chain or a branched chain having 2 to 5 carbon atoms.

The polymerizable compound represented by Chemical formula B easily synthesized by, for example, by the following method including Suzuki-Miyaura coupling reaction. The polymerizable compound represented by Chemical formula B is a new compound. However, when the number of carbon atoms in X is two, a phenyl group having R₁ to R₅ groups is different from a phenyl group having R₁' to R₅' groups.

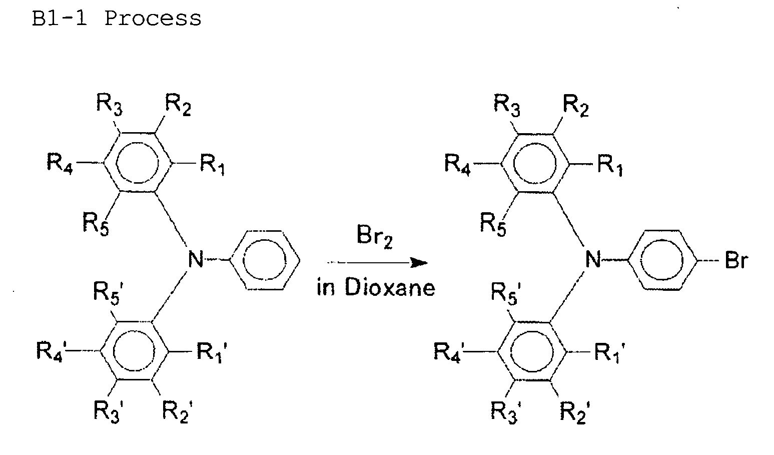

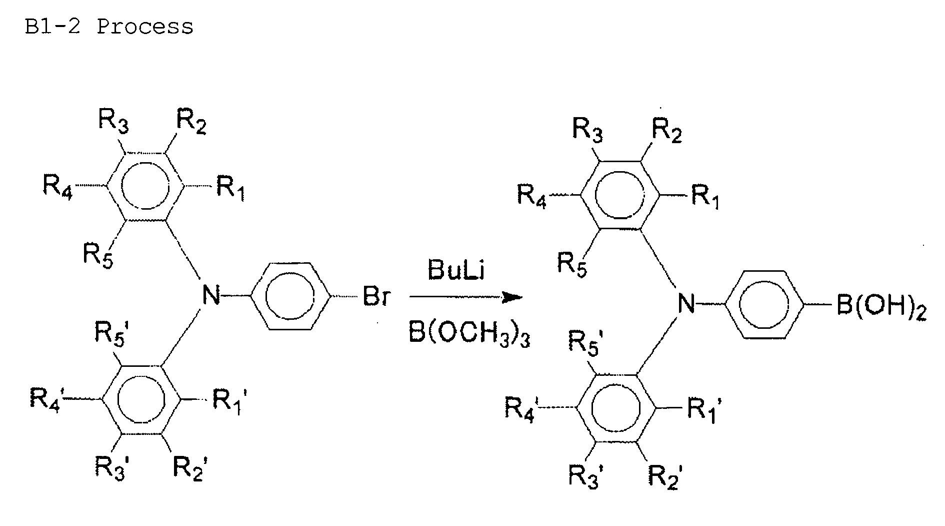

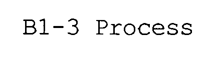

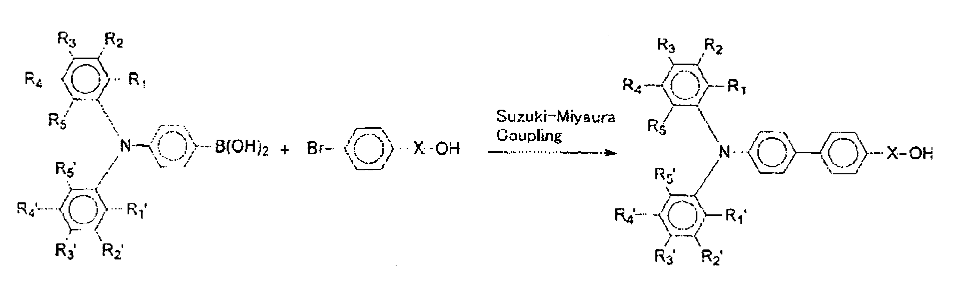

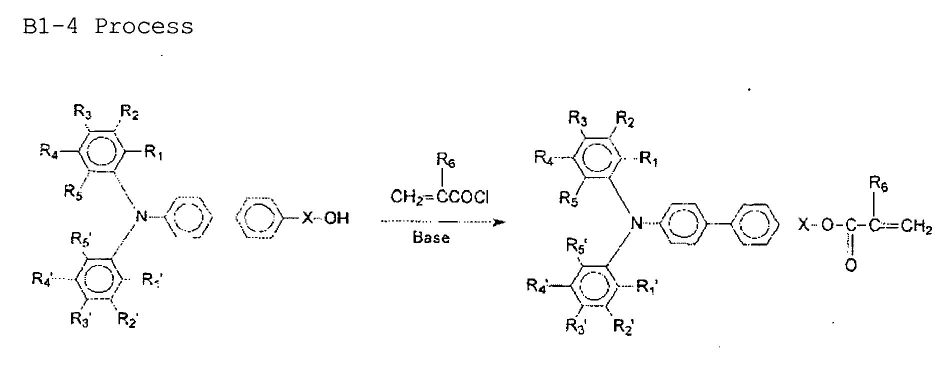

Synthesis of the intermediate can be changed as follows:

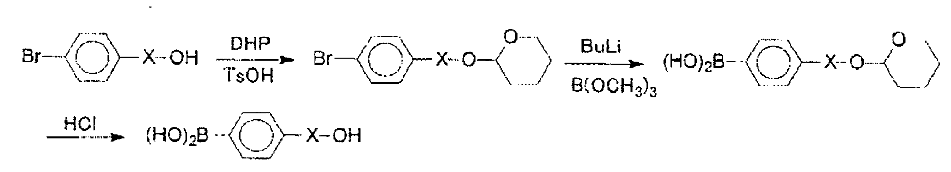

In the process, DHP represents 3,4-dihydro-2H-pyrane and TSOH represents paratoluene sulfonic acid.

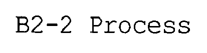

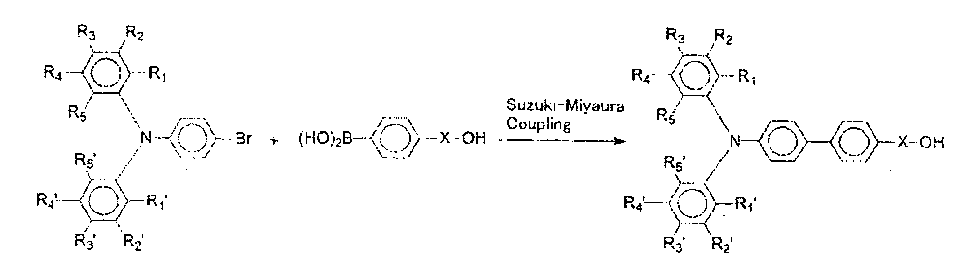

Specific examples of the radical reactive compounds represented by Chemical formula B includes, but are not limited to, the following:

| | | | | | | | | | | | | |
|---|---|---|---|---|---|---|---|---|---|---|---|---|
| 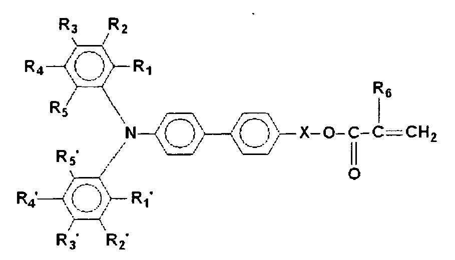 | | | | | | | | | | | | |

| No. | R₁ | R₂ | R₃ | R₄ | R₅ | R₁' | R₂' | R₃' | R₄' | R₅' | R₆ | -X- |
|---|---|---|---|---|---|---|---|---|---|---|---|---|
| 1 | H | H | H | H | H | H | H | H | H | H | H | -CH₂CH₂- |
| 2 | H | H | CH₃ | H | H | H | H | H | H | H | H | -CH₂CH₂- |
| 3 | H | H | CH₃ | H | H | H | H | CH₃ | H | H | H | -CH₂CH₂- |
| 4 | CH₃ | H | CH₃ | H | H | H | H | H | H | H | H | -CH₂CH₂- |
| 5 | H | CH₃ | CH₃ | H | H | H | H | H | H | H | H | -CH₂CH₂- |
| 6 | CH₃ | H | CH₃ | H | CH₃ | H | H | H | H | H | H | -CH₂CH₂- |
| 7 | CH₃ | CH₃ | H | H | H | H | H | H | H | H | H | -CH₂CH₂- |
| 8 | CH₃ | H | CH₃ | H | H | H | H | CH₃ | H | H | H | -CH₂CH₂- |
| 9 | H | CH₃ | CH₃ | H | H | H | H | CH₃ | H | H | H | -CH₂CH₂- |
| 10 | CH₃ | H | CH₃ | H | CH₃ | H | H | CH₃ | H | H | H | -CH₂CH₂- |
| 11 | CH₃ | CH₃ | H | H | H | H | H | CH₃ | H | H | H | -CH₂CH₂- |
| 12 | CH₃ | H | CH₃ | H | H | CH₃ | H | CH₃ | H | H | H | -CH₂CH₂- |
| 13 | H | CH₃ | CH₃ | H | H | CH₃ | H | CH₃ | H | H | H | -CH₂CH₂- |
| 14 | CH₃ | H | CH₃ | H | CH₃ | CH₃ | H | CH₃ | H | H | H | -CH₂CH₂- |
| 15 | CH₃ | CH₃ | H | H | H | CH₃ | H | CH₃ | H | H | H | -CH₂CH₂- |
| 16 | H | CH₃ | CH₃ | H | H | H | CH₃ | CH₃ | H | H | H | -CH₂CH₂- |
| 17 | CH₃ | H | CH₃ | H | CH₃ | H | CH₃ | CH₃ | H | H | H | -CH₂CH₂- |
| 18 | CH₃ | CH₃ | H | H | H | H | CH₃ | CH₃ | H | H | H | -CH₂CH₂- |
| 19 | CH₃ | H | CH₃ | H | CH₃ | CH₃ | H | CH₃ | H | CH₃ | H | -CH₂CH₂- |

**Table 1-2**

| No. | R₁ | R₂ | R₃ | R₄ | R₅ | R₁' | R₂' | R₃' | R₄' | R₅' | R₆ | -X- |
|---|---|---|---|---|---|---|---|---|---|---|---|---|
| 20 | H | H | H | H | H | H | H | H | H | H | CH₃ | -CH₂CH₂- |
| 21 | H | H | CH₃ | H | H | H | H | H | H | H | CH₃ | -CH₂CH₂- |
| 22 | H | H | CH₃ | H | H | H | H | CH₃ | H | H | CH₃ | -CH₂CH₂- |
| 23 | CH₃ | H | CH₃ | H | H | H | H | CH₃ | H | H | CH₃ | -CH₂CH₂- |
| 24 | H | CH₃ | CH₃ | H | H | H | H | CH₃ | H | H | CH₃ | -CH₂CH₂- |
| 25 | CH₃ | H | CH₃ | H | CH₃ | H | H | CH₃ | H | H | CH₃ | -CH₂CH₂- |
| 26 | H | H | CH₃ | H | H | H | H | CH₃ | H | H | H | -CH₂CH₂CH₂- |
| 27 | CH₃ | H | CH₃ | H | H | H | H | CH₃ | H | H | H | -CH₂CH₂CH₂- |
| 28 | H | CH₃ | CH₃ | H | H | H | H | CH₃ | H | H | H | -CH₂CH₂CH₂- |
| 29 | CH₃ | H | CH₃ | H | CH₃ | H | H | CH₃ | H | H | H | -CH₂CH₂CH₂- |
| 30 | H | H | CH₃ | H | H | H | H | CH₃ | H | H | H | -CH₂CH₂CH₂CH₂- |
| 31 | CH₃ | H | CH₃ | H | H | H | H | CH₃ | H | H | H | -CH₂CH₂CH₂CH₂- |
| 32 | H | CH₃ | CH₃ | H | H | H | H | CH₃ | H | H | H | -CH₂CH₂CH₂CH₂- |
| 33 | CH₃ | H | CH₃ | H | CH₃ | H | H | CH₃ | H | H | H | -CH₂CH₂CH₂CH₂- |
| 34 | CH₃ | H | CH₃ | H | CH₃ | CH₃ | H | CH₃ | H | CH₃ | H | -CH₂CH₂CH₂CH₂- |
| 35 | H | H | CH₃ | H | H | H | H | CH₃ | H | H | H | -CH₂CH₂CH₂CH₂CH₂- |
| 36 | CH₃ | H | CH₃ | H | H | H | CH₃ | CH₃ | H | H | H | -CH₂CH₂CH₂CH₂CH₂- |
| 37 | CH₃ | CH₃ | H | H | H | H | CH₃ | CH₃ | H | H | H | -CH₂CH₂CH₂CH₂CH₂- |
| 38 | CH₃ | H | CH₃ | H | CH₃ | CH₃ | CH₃ | H | H | H | H | -CH₂CH₂CH₂CH₂CH₂- |

Table 1-3

**Table 1-3**

| No. | R₁ | R₂ | R₃ | R₄ | R₅ | R₁' | R₂' | R₃' | R₄' | R₅' | R₆ | -X- |
|---|---|---|---|---|---|---|---|---|---|---|---|---|
| 39 | H | H | CH₃ | H | H | H | H | CH₃ | H | H | H | 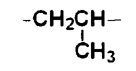 |
| 40 | H | H | CH₃CH₂ | H | H | H | H | CH₃ | H | H | H | 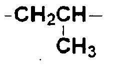 |
| 41 | H | H | CH₃CH₂ | H | H | H | H | CH₃CH₂ | H | H | CH₃ | 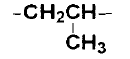 |
| 42 | CH₃ | H | H | H | CH₃CH₂ | H | H | H | H | H | H | 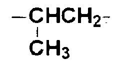 |
| 43 | H | H | CH₃ | H | H | H | H | CH₃ | H | H | CH₃ | 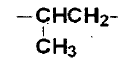 |
| 44 | H | H | 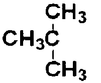 | H | H | H | H | CH₃ | H | H | H | 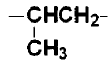 |
| 45 | CH₃ | H | CH₃ | H | CH₃ | H | H | CH₃CH₂O | H | H | H | 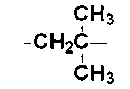 |
| 46 | H | H | CH₃O | H | H | H | H | CH₃O | H | H | H | 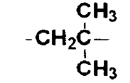 |
| 47 | H | H | CH₃O | H | H | H | H | CH₃ | H | H | H | 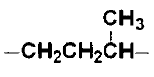 |
| 48 | H | H | 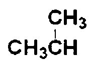 | H | H | CH₃ | H | CH₃ | H | H | CH₃ | 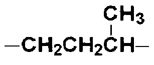 |
| 49 | H | H | CH₃ | H | H | H | H | CH₃ | H | H | H | 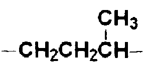 |
| 50 | CH₃ | H | CH₃ | H | H | CH₃ | H | CH₃ | H | H | H | 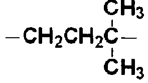 |
| 51 | CH₃ | H | CH₃ | H | CH₃ | CH₃ | H | CH₃ | H | CH₃ | H | 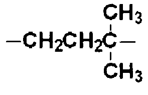 |

The content of the radical reactive compound represented by Chemical formula B suitably used for the present invention is from 20 to 80 % by weight and preferably from 30 to 70 % by weight based on the total weight of the composition having curing property. When the content of the radical polymerizable compound is too small, the structure unit represented by Chemical formula A tends to be thin in the cured composition so that it is not possible to improve the charge transport property for a thickened layer and restrain image deterioration caused by environmental change and voltage variation due to oxidized gas. When the content of the radical polymerizable compound is too large, the number of curing function groups is in sufficient so that the mechanical strength such as anti-abrasion and anti-damage may deteriorate.

### Monomer Having Three Or More Radical Polymerizable function groups

The cross linking surface layer for use in the present invention includes a cured composition having the structure unit represented by Chemical formula A. Optionally, a monomer having three or more radical polymerizable function groups can be contained in the cross linking surface layer for adjusting the anti-abrasion property, hardness, viscosity of liquid of application, curing speed, etc. Specific examples of the radical polymerizable function groups include known radical polymerizable function groups and especially acryloyloxy groups and methacryloyloxy groups are preferred.

Specific examples of the radical polymerizable monomers having three or more function groups include, but are not limited to, the following compounds:
Specific suitable examples of the monomer having three or more radical polymerizable function groups for use in the present invention include, but are not limited to, trimethylolpropane triacrylate (TMPTA), trimethylolpropane trimethacrylate, HPA modified trimethylolpropane triacrylate, trimethylol propane ethylene oxy-modified (EO-modified) triacrylate, trimethylolpropane propyleneoxy-modified (PO-modified) triacrylate, trimethylolpropane caprolactone-modified triacrylate, trimethylolpropane HPA-modified trimethacrylate, pentaerythritol triacrylate, pentaerythritol tetraacrylate (PETTA), glycerol triacrylate, glycerol epichlorohydrine-modified (ECH-modified) triacrylate, glycerol EO-modified triacrylate, glycerol PO-modified triacrylate, tris(acryloxyethyl)isocyanurate, dipentaerythritol hexaacrylate (DPHA), dipentaerythritol caprolactone-modified hexaacrylate, dipentaerythritol hydroxypentaacrylate, alkyl-modified dipentaerythritol pentaacrylate, alkyl-modified dipentaerythritol tetraacrylate, alkyl-modified dipentaerythritol triacrylate, dimethylolpropane tetraacrylate (DTMPTA), pentaerythritol ethoxytetraacrylate, phosphoric acid EO-modified triacrylate, and 2,2,5,5-tetrahydroxymethyl cyclopentanone tetraacrylate. These can be used alone or in combination.

The content of the monomer having three or more radical polymerizable groups for use in the present invention is from 0 to 90 % by weight and preferably from 0 to 50 % by weight based on the total weight of the composition having a curing property. In light of the effect of the present invention, it is preferred to restrain the content of the monomer within a range of not greater than the content of the radical polymerizable reactive compound represented by Chemical formula B.

### Other Additive having Radical Polymerizable function groups

The cross linking surface layer for use in the present invention includes a cured composition having the structure unit represented by Chemical formula A. Radical polymeriable monomers having one or two function groups, functional monomers and radical polymerizable oligomers can be contained in the cross linking surface layer in combination for imparting functions such as adjusting viscosity during application, relaxing the stress of the cross linking surface layer, lowering the surface energy and reducing the friction index.
Compounds known as the radical polymerizable monomers and oligomers can be used.

Specific examples of the radical polymerizable monomer having one function group include, but are not limited to, 2-ethylhexyl acrylate, 2-hydroxyethyl acrylate, 2-hydroxypropyl acrylate, tetrahydrofurfuryl acrylate, 2-ethylhexyl carbitol acrylate, 3-methoxybutyl acrylate, benzyl acrylate, cyclohexyl acrylate, isoamyl acrylate, isobutyl acrylate, methoxy triethylene glycol acrylate, phenoxy tetraethylene glycol acrylate, cetyl acrylate, isostearyl acrylate, stearyl acrylate and styrene.

Specific examples of the radical polymerizable monomer having two function groups include, but are not limited to, 1,3-butandiol diacrylate, 1,4-butane diol diacrylate, 1,4-butane diol dimethacrylate, 1,6-hexane diol diacrylate, 1,6-hexane diol dimethacrylate, diethylene glycol diacrylate, neopenthyl glycol diacrylate, bisphenol A - EO modified diacrylate, bisphenol F - EO modified diacrylate and neopentyl glycol diacrylate.

Specific examples of the functional monomers include, but are not limited to, monomers in which a fluorine atom of, for example, octafluoro penthyl acrylate, 2-perfluorooctyl ethyl acrylate, 2-perfluorooctyl ethyl methacrylate and 2-perfluoroisononyl ethyl acrylate is substituted, and vinyl monomers, acrylates and methacrylates having polysiloxane groups, for example, acryloyl polydimethyl siloxane ethyl, methacryloyl polydimethyl siloxane ethyl, acryloyl polydimethyl siloxane propyl, acryloyl polydimethyl siloxane butyl and diacryloyl polydimethyl siloxane diethyl having 20 to 70 siloxane repeating units set forth in examined published Japanese patent applications No. (hereinafter referred to as JPPs) H05-60503 and H06-45770.

Specific examples of the radical polymerizable oligomers include, but are not limited to, epoxyacrylate based, urethane acrylate based, and polyester acrylate based oligomers.

When a radical polymerizable monomer and/or a radical polymerizable oligomer having one or two function groups are contained in a large amount, the three dimensional cross linking density of the crosslinked surface layer substantially decreases, which invites the deterioration of the anti-abrasion property. Therefore, the content of these monomers and oligomers is limited to 30 % by weight and more preferably 20 % by weight based on the total weight of the composition having a curing property of the crosslinked surface layer.

### Polymerization Initiator

The crosslinked surface layer for use in the present invention includes a cured composition having the structure unit represented by Chemical formula (A). To effectively conduct the curing reaction, a polymerization initiator can be contained in a liquid application for the crosslinked surface layer, if desired.

Specific examples of photo polymerization initiators include, but are not limited to, acetophenone based or ketal based photo polymerization initiators, for example, diethoxy acetopenone, 2,2-dimethoxy-1,2-diphenylethane-1-one, 1-hydroxy cyclohexyl phenylketone, 4-(2-hydroxyethoxy)phenyl-(2-hydroxy-2-propyl)ketone, 2-benzyl-2-dimethylamino-1-(4-morpholinophenyl)butanone-1,2-hydroxy-2-methyl-1-phenylpropane-1-one, 2-methyl-2-morpholino(4-methylthiophenyl)propane-1-one, and 1- phenyl-1,2-propane dione-2-(o-ethoxycarbonyl)oxime; benzoin ether based photo polymerization initiators, for example, benzoine, benzoine methyl ether, benzoin ethyl ether, benzoine isobutyl ether and benzoine isopropyl ether; benzophenone based photo polymerization initiators, for example, benzophenone, 4-hydroxy benzophenone, o-benzoyl benzoic acid methyl, 2-benzoyl naphthalene, 4-benzoyl biphenyl, 4-benzoyl phenyl ether, acrylated benzophenone and 1, 4-benzoyl benzene; and thioxanthone based photo polymerization initiators, for example, 2-isopropyl thioxanthone, 2-chloro thioxanthone, 2,4-dimethyl thioxanthone, 2,4-diethyl thioxanthone, and 2,4-dichloro thioxanthone.

Other photo polymerization initiators are, for example, ethylanthraquinone, 2,4,6-trimethyl benzoyl diphenyl phosphine oxide, 2,4,6-trimethyl benzoyl phenyl ethoxy phosphine oxide, bis(2,4,6-trimethyl benzoyl)phenyl phosphine oxide, bis(2,4-dimethoxy benzoyl)-2,4,4-trimethyl pentyl phosphine oxide, methylphenyl glyoxy esters, 9,10-phenanthrene, acridine based compounds, triadine based compounds, and imidazole based compounds. In addition, compounds having photo polymerization promotion effect can be used alone or in combination with the photo polymerization initiators mentioned above. Specific examples thereof include triethanol amine, methyldiethanol amine, 4-dimethylamino ethyl benzoate, 4-dimethylamino isoamile benzoate, benzoic acid (2-dimethylamino)ethyl, and 4,4'-dimethylamino benzophenone.

Specific examples of thermal polymerization initiator include, but are not limited to, peroxide-based initiators, for example, 2,5-dimethylhexane-2,5-dihydroperoxide, dicumyl peroxide, benzoyl peroxide, t-butyl cumyl peroxide, 2,5-dimethyl-2,5-di(peroxybenzoyl)hexyne-3,di-t-butyl peroxide, t-butylhydroperoxide, cumene hydroperoxide, lauroyl peroxide and 2,2-bis(4,4-di-t-butyl peroxy cyclohexy)propane, and azo based initiators, for example, azobis isobutylnitrile, azobiscyclohexane carbonitrile, azobis methyl isobutyric acid, azobis isobutyl amidine hydrochloride salts, and 4,4'-azobis-4-cyano valeric acid.

These polymerization initiators can be used alone or in combination. The addition amount of the polymerization initiator is from 0.5 to 40 parts by weight and preferably from 1 to 20 parts by weight based on 100 parts by weight of the total weight of the radical polymeric compound.

### Other Additives

Furthermore, a liquid application for the cross linking surface layer can contain other additives, for example, various kinds of a plasticizing agent to relax stress and improve adhesibility, a leveling agent, and a low molecular weight charge transport material which is not radical polymeric, if desired. Known additives can be used. Specific examples of the plasticizing agent include compounds, for example, dibutyl phthalate and dioctyl phthalate, which are used for typical resins. The addition amount of the plasticizing agent is not greater than 20 % by weight and more preferably not greater than 10 % by weight based on all the solid portion of the liquid of application. Specific examples of the leveling agent include silicone oils, for example, dimethyl silicone oil and methyl phenyl silicone oil, and polymers or oligomers having perfluoroalkyl groups in its side chain. The addition amount of the leveling agent is not greater than 3 % by weight based on the total solid portion of the liquid application. Low molecular weight charge transport materials are good to improve the charge transportability of a cross linking surface layer and decrease the irradiation voltage but have a trade-off relationship in light of the anti-abrasion because the content of cured material decreases due to the addition of such a low molecular weight charge transport materials. Thereby, although the addition amount thereof is adjusted depending on the process in which such a low molecular weight is used, it is not greater than 50 % by weight and preferably not greater than 30 % by weight based on all the components of a crosslinked surface layer. Binder resins are effective in decreasing the internal stress in a crosslinked surface layer, thereby improving uniformity, and reducing the occurrence of cracking and damage. However, as in the case of the other additives, the addition of a binder resin has an adverse impact on the anti-abrasion property and consequently the addition amount thereof is limited to not greater than 20 % by weight and preferably not greater than 10 % by weight.

### Solvent of Liquid application

The crosslinked surface layer for use in the present invention is formed by applying a liquid application containing at least a radical reactive compound having a structure unit of Chemical formula A to a photosensitive layer, which is described later, followed by curing. When the radical reactive compound is liquid, the other components is dissolved therein for application. If desired, a solvent is used to dilute these before application. Specific examples of such solvents include, but are not limited to, an alcohol based solvent, such as methanol, ethanol, propanol and butanol; a ketone based solvent, such as acetone, methyl ethyl ketone, methyl isobutyl ketone, and cyclohexanone; an ester based solvent, such as ethyl acetate and butyl acetate; an ether based solution, such as tetrahydrofuran dioxane and propyl ether; a halogen based solvent, such as dichloromethane, dichloroethane, trichloroethane and chlorobenzene; an aromatic series based solvent, such as benzene, toluene and xylene; and a cellosolve based solvent, such as methyl cellosolve, ethyl cellosove and cellosolve acetate. These solvents can be used alone or in combination. The dilution ratio by these solvents depends on the solubility and the coating method of a composition, and a desired layer thickness. A dip coating method, a spray coating method, a beat coating method, a ring coating method, etc., can be used for coating the liquid application.

In the present invention, subsequent to the application of a liquid application for the crosslinked surface layer, a crosslinked surface layer is cured upon application of external energy, for example, heat, light and radiation ray. As light energy, a UV irradiation light source, for example, a high pressure mercury lamp or a metal halide lamp having an emission wavelength mainly in the ultraviolet area can be used. A visible light source can be selected according to the absorption wavelength of a radical polymeric compound and a photopolymerization initiator. The irradiation light amount is preferably from 50 mW/cm² to 1,000 mW/cm². When the irradiation light amount is too small, it takes a long time to complete the curing reaction. When the irradiation light amount is too large, the reaction is not uniformly performed, resulting in the occurrence of wrinkle on the surface of the crosslinked surface layer and a significant amount of non-reacted groups and polymerization terminated ends. In addition, the internal stress in the surface layer increases due to such rapid cross-linking, which causes cracking and peeling thereof. The irradiation time depends on the penetration degree of UV light determined by composition and layer thickness and is preferably from 5 seconds to 5 minutes. When the irradiation time is too short, curing tends to be insufficient. When the irradiation time is too long, the material composition is decomposed, resulting in deterioration of electric characteristics. In addition, it is preferred to control the temperature rise during curing within 50 °C to prevent decomposition of the material composition and reduce the occurrence of non-uniform curing reaction.

When the thermal energy is used for curing, the curing time is preferably from 10 minutes to 3 hours at a temperature range of from 100 to 170 °C. When the temperature is too low or the curing time is too short, curing tends to be insufficient. When the temperature is too high or the curing time is too long, the material composition easily decomposes and non-uniform curing reaction is performed, which leads to formation of an unsuitable crosslinked surface layer.

As the irradiation energy, electron beam is suitably used. The liquid application is cured under the conditions that the acceleration voltage of an electron beam is not greater than 300 KV, and preferably not greater than 150 KV and the radiation dose thereof is from 1 to 100 Mrad. When the acceleration voltage is too high or the radiation dose is too high, the material composition easily decomposes so that the effect of the present invention is not obtained.

Among these forms of energies, thermal or light energy is suitably used in terms of easiness of reaction speed control and simplicity of a device.

The layer thickness of the crosslinked surface layer for use in the present invention is different depending on the structure of a photosensitive layer, which is located under the crosslinked surface layer and not cured, and is described later at the time when the description about the structure of the photosensitive layer is made. The present invention is described according to the layer structure.

### Layer Structure of Image Bearing Member

The image bearing member of the present invention is described with reference to the accompanying drawings.

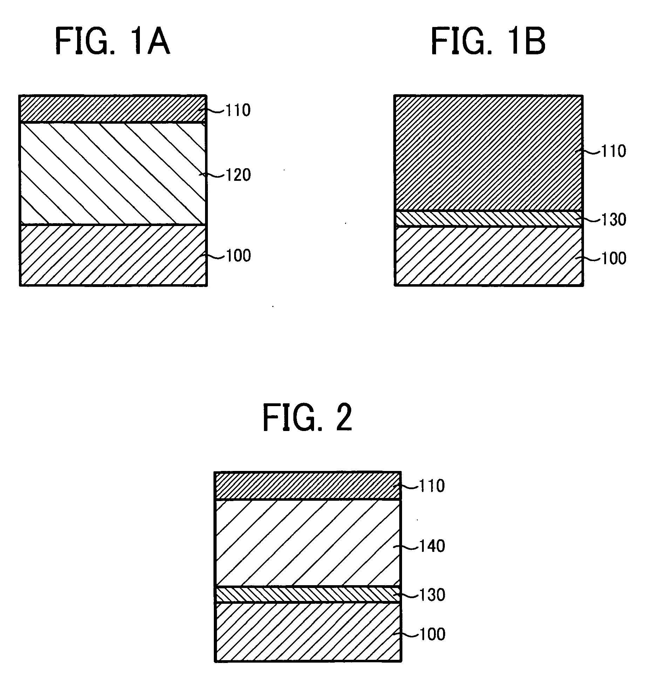

Figs. 1 and 2 are cross sections illustrating examples of the image bearing member of the present invention.
Fig. 1A is a single layer structure in which a photosensitive layer 120 having a charge generation function and a charge transport function simultaneously is provided on an electroconductive substrate 100 and a crosslinked surface layer 110 is the surface portion of the photosensitive layer 120. That is, the photosensitive layer 120 having a charge generation function represents the uncured photosensitive layer and a charge transport function simultaneously and the crosslinked surface layer 110 represents the crosslinked surface layer having a curing compound.
Fig. 1B is a single layer structure in which a charge generation layer 130 having a charge generation function is provided on the electroconductive substrate 100 as the photosensitive layer and the crosslinked surface layer 110 including a charge transport compound represents the surface portion of the photosensitive layer. That is, the charge transport layer 130 represents the uncured photosensitive layer and the crosslinked surface layer 110 represents the crosslinked surface layer having a curing compound.
Fig. 2 is a diagram illustrating an examples of a multiple layer structured image bearing member of the present invention in which the charge generation layer 130 having a charge generation function and a charge transport layer 140 having a charge transport function are accumulated on the electroconductive substrate 100 as a photosensitive layer. That is, the charge generation layer 130 and the charge transport layer 140 correspond to the uncured photosensitive layer and the crosslinked surface layer 110 corresponds to the crosslinked surface layer having a curing compound.

### Electroconductive Substrate

Materials having a volume resistance of not greater than 10¹⁰ Ω·cm can be used as a material for the electroconductive substrate 100. For example, there can be used plastic or paper having a film form or cylindrical form covered with a metal, such as aluminum, nickel, chrome, nichrome, copper, gold, silver, and platinum, or a metal oxide, chrome, nichrome, copper, gold, silver, and platinum, or a metal oxide, such as tin oxide and indium oxide by depositing or sputtering. Also a board formed of aluminum, an aluminum alloy, nickel, and a stainless metal can be used. Further, a tube which is manufactured from the board mentioned above by a crafting technique, for example, extruding and extracting, and surface-treatment, such as cutting, super finishing and grinding, is also usable. In addition, an endless nickel belt and an endless stainless belt described in JOP S52-36016 can be used as the electroconductive substrate.

An electroconductive substrate can be also formed by applying to the substrate mentioned above a liquid application in which electroconductive powder is dispersed in a suitable binder resin and can be used as the electroconductive substrate for use in the present invention.

Specific examples of such electroconductive powders include, but are not limited to, carbon black, acetylene black, metal powder, such as powders of aluminum, nickel, iron, nichrome, copper, zinc and silver, and metal oxide powder, such as electroconductive tin oxide powder and ITO powder.

Specific examples of the binder resins which are used together with the electroconductive powder include, but are not limited to, thermoplastic resins, thermosetting resins, and optical curing resins, such as a polystyrene, a styrene-acrylonitrile copolymer, a styrene-butadiene copolymer, a styrene-anhydride maleic acid copolymer, a polyester, a polyvinyl chloride, a vinyl chloride-vinyl acetate copolymer, a polyvinyl acetate, a polyvinylidene chloride, a polyarylate (PAR) resin, a phenoxy resin, polycarbonate, a cellulose acetate resin, an ethyl cellulose resin, a polyvinyl butyral, a polyvinyl formal, a polyvinyl toluene, a poly-N-vinyl carbazole, an acrylic resin, a silicone resin, an epoxy resin, a melamine resin, an urethane resin, a phenol resin, and an alkyd resin. Such an electroconductive layer can be formed by dispersing the electroconductive powder and the binder resins mentioned above in a suitable solvent, for example, tetrahydrofuran (THF), dichloromethane (MDC), methyl ethyl ketone (MEK), and toluene and applying the resultant to an electroconductive substrate.

In addition, an electroconductive substrate formed by providing a heat contraction tube as an electroconductive layer on a suitable cylindrical substrate can be used as the electroconductive substrate in the present invention. The heat contraction tube can be formed of a material, such as polyvinyl chloride, polypropylene, polyester, polystyrene, polyvinylidene chloride, polyethylene, chloride rubber, and TEFLON® in which the electroconductive powder mentioned above is contained.

### Photosensitive Layer

The photosensitive layer is described. The photosensitive layer has a laminate structure or a single layer structure.

In the case of a laminate structure, the photosensitive layer is formed of a charge generating layer having a charge generation function and a charge transport layer having a charge transport function. In the case of a single layer structure, the photosensitive layer is a layer having a charge generating function and a charge transport function simultaneously or a layer having a charge generating layer.

Below is a description about both a photosensitive layer having a laminate structure and a photosensitive layer having a single layer structure.

### Photosensitive Layer Having Laminate Strcuture

### (1) Charge Generation Layer

The charge generation layer is a layer mainly formed of a charge generation material having a charge generation function and can include a binder resin, if desired. Inorganic materials and organic materials can be used as the charge generation material.

Specific examples of the inorganic materials include crystal selenium, amorphous selenium, selenium-tellurium, selenium-tellulium-halogne, selenium-arsenic compounds and amorphous silicon. With regard to the amorphous silicon, amorphous silicon in which the dangling bonding is terminated by hydrogen atoms and halogen atoms or boron atoms and phosphorous atoms are doped are suitably used.

On the other hand, known materials can be used as the organic materials. Specific examples thereof include phthalocyanine based pigments, for example, metal phthalocyanine and non-metal phthalocyanine, azulenium salt pigments, methine squaric acid pigments, azo pigments having carbazolee skeleton, azo pigments having triphenyl amine skeleton, azo pigments having dibenzothiophene skeleton, azo pigments having fluorenone skeleton, azo pigments having oxadiazole skeleton, azo pigments having bis stilbene skeleton, azo pigments having distyryl oxadiazole skeleton, azo pigments having distyryl carbazolee skeleton, perylene based pigments, anthraquinone based or polycyclic quinone pigments, quinone imine pigments, diphenyl methane based pigments, triphenyl methane based pigments, benzoquinone based pigments, naphthoquinone based pigments, cyanine based pigments, azomethine based pigments, indigoid based pigments, and bisbenzimidazole pigments. These charge generating materials can be used alone or in combination.

Specific examples of the binder resin optionally used in the charge generation layer include, but are not limited to, polyamide resins, polyurethane resins, epoxy resins, polyketone resins, polycarbonate resins, silicone resins, acryl resins, polyvinyl butyral resins, polyvinyl formal resins, polyvinyl ketone resins, polystyrene resins, poly(N-vinylcarbazole) resins, and polyacrylamide resins. These binder resins can be used alone or in combination. As the binder reins for the charge generation layer, in addition to the binder resins mentioned above, charge transport polymer materials having a charge transport function, for example, polymer materials, for example, polycarbonate resins, polyester resins, polyurethane resins, polyether resins, polysiloxane resins, and acryl resins which have arylamine skeleton, benzidine skeleton, hydrazone skeleton, carbazolee skeleton, stilbene skeleton, pyrazoline skeleton, etc.; and polymer materials having polysilane skeleton, can be used.

Specific examples of the charge transport polymers include compounds described in JOPs H01-001728, H01-009964, H01-013061, H01-019049, H01-241559, H04-011627, H04-175337, H04-183719, H04-225014, H04-230767, H04-320420, H05-232727, H05-310904, H06-234836, H06-234837, H06-234838, H06-234839, H06-234840, H06-234840, H06-234841, H06-239049, H06-236050, H06-236051, H06-295077, H07-056374, H08-176293, H08-208820, H08-211640, H08-253568, H08-269183, H09-062019, H09043883, H09-71642, H09-87376, H09-104746,H09-110974, H09-110974, H09-110976, H09-157378, H09-221544, H09-227669, H09-221544, H09-227669, H09-235367, H09-241369, H09-268226, H09-272735, H09-272735, H09-302084, H09-302085 and H09-328539.

Specific examples of the latter charge transport polymers include polysiylene polymers described in JOPs S63-285552, H05-19497, H05-70595 and H10-73944.

The charge generation layer can contain a charge transport material having a low molecular weight.

There are two types of the charge transport materials which can be used for the charge generation layer. These are positive hole transport materials and electron transport materials.

Specific examples of such electron transport materials include, but not limited to, electron acceptance materials such as chloranil, bromanil, tetracyano ethylene, tetracyanoquino dimethane, 2,4,7-trinitro-9-fluorenone, 2,4,5,7-tetranitro-9-fluorenone, 2,4,5,7-tetranitroxanthone, 2,4,8-trinitrothioxanthone, 2,6,8-trinitro-4H-indeno[1,2-b]thiophene-4-one, 1,3,7-trinitrodibenzothhiophene-5,5-dioxide, and diphenoquinone derivatives.

These electron transport materials can be used alone or in combination.

Specific examples of such positive hole transport materials include, but not limited to, oxazole derivatives, oxadiazole derivatives, imidazole derivatives, monoaryl amine derivatives, diaryl amine derivatives, triaryl amine derivatives, stilbene derivatives, α-phenyl stilbene derivatives, benzidine derivatives, diaryl methane derivatives, triaryl methane derivatives, 9-styryl anthracene derivatives, pyrazoline derivatives, divinyl benzene derivatives, hydrazone derivatives, indene derivatives, butadiene derivatives, pyrene derivatives, bisstilbene derivatives, enamine derivatives and other known materials. These positive hole transport materials can be used alone or in combination.

As a method of forming a charge generating layer, it is possible to use a vacuum thin layer manufacturing method and a casting method from a solution dispersion system.

Specific examples of the vacuum thin layer manufacturing method include, but not limited to, a vacuum deposition method, a glow discharging decomposition method, an ion plating method, a sputtering method, and a reactive sputtering method and a chemical vacuum deposition (CVD) method. Both inorganic materials and organic materials can be used for forming a charge transport layer.

When a casting method is used, if desired, it is possible to form a charge generating layer by applying a suitably diluted liquid dispersion obtained by dispersing the inorganic material or the organic material mentioned above in a solvent together with a binder resin using a dispersion device. Specific examples of the solvent include, but not limited to, tetrahydrofuran, dioxane, dioxolan, toluene, dichloromethane, monochlorobenzene, dichloroethane, cyclohexanone, cyclopentanone, anisole, xylene, methylethylketone, acetone, ethyl acetate and butyl acetate. Specific examples of the dispersing device include, but not limited to, a ball mill, an attritor, a sand mill, and a bead mill. In addition, if desired, a leveling agent, for example, dimethyl silicone oil and methylphenyl silicone oil, can be added to the liquid dispersion mentioned above. Furthermore, the application mentioned above is performed by a dip coating method, a spray coating method, a bead coating method and a ring coating method.

In the present invention, the thickness of the charge generating layer is preferably from 0.01 to 5 µm and more preferably from 0.05 to 2 µm.

### (2) Charge Transport Layer

The charge transport layer is a layer having a charge transport function. The charge transport layer is formed by dissolving and/or dispersing a charge transport material and a binder resin in a suitable solvent and applying the liquid to the charge generation layer followed by drying.

The electron transport materials, the positive hole transport materials and charge transport polymer mentioned above in the description about the charge generating layer can be used as the charge transport material. As described above, by using a charge transport polymer, it is possible to reduce the solubility of the underlayer when a surface layer is coated, which is especially useful.

Specific examples of the binder resin include, but not limited to, thermoplastic resins or thermocuring resins, for example, polystyrene, copolymers of styrene and acrylonitrile, copolymers of styrene and butadiene, copolymers of styrene and maleic anhydrate, polyesters, polyvinyl chlorides, copolymers of a vinyl chloride and a vinyl acetate, polyvinyl acetates, polyvinylidene chloride, polyarylate resins, phenoxy resins, polycarbonate reins, cellulose acetate resins, ethyl cellulose resins, polyvinyl butyral, polyvinyl formal, polyvinyl toluene, poly-N-vinylcarbozole, acrylic resin, silicone resins, epoxy resins, melamine resins, urethane resins, phenol resins, and alkyd resins.

The content of the charge transport material is from 20 to 300 parts by weight and preferably from 40 to 150 parts by weight based on 100 parts by weight of the binder resin. When a charge transport polymer is used, it is possible to use such a charge transport polymer alone or in combination with a binder resin.

As a solvent for use in application of the charge transport layer, the same as the solvents for the charge generation layer can be used. Solvents that suitably dissolve a charge transport material and a binder resin are preferred. These solvents can be used alone or in combination. It is also possible to use the same method in the case of the charge generation layer for forming the charge transport layer.

In the present invention, a plasticizing agent and/or a leveling agent can be contained, if desired.

Specific examples of the plasticizing agent for use in the charge transport layer include, but not limited to, dibutyl phthalate and dioctyl phthalate, which are used for typical resins. The addition amount of the plasticizing agent is preferably from 0 to 30 parts by weight based on 100 parts by weight of a binder resin.

Specific examples of the leveling agent for use in the charge transport layer include, but not limited to, silicone oils, for example, dimethyl silicone oil and methyl phenyl silicone oil, and polymers or oligomers having perfluoroalkyl groups in its side chain. The addition amount of the leveling agent is preferably from 0 to 1 parts by weight based on 100 parts by weight of a binder resin.

The layer thickness of the charge transport layer is suitably from about 5 to about 40 µm and preferably from about 10 to about 30 µm.

As described in the method of manufacturing the cross linking surface layer, the cross linking surface layer is formed by applying the liquid application for the cross linking surface layer for use in the present invention to the charge transport layer followed by optional drying and starting the curing reaction upon irradiation of light, heat, or radiation. The layer thickness of the cross linking surface layer is from 1 to 15 µm and preferably from 2 to 13 µm. When the layer thickness is too thick, cracking and layer peeling tend to occur. In addition, deterioration of the sensitivity tends to be significant. When the layer thickness is too thin, the durability is easily affected depending on the thickness.

### Single layered Photosensitive Layer

The single layered photosensitive layer is a layer having both functions of charge generation function and charge transport function simultaneously or a charge generation layer having a charge generation function.

### Single Layered Photosensitive Layer Having Both Functions of Charge Generation Function and Charge Transport Function Simultaneously

When the photosensitive layer is a layer having both functions of charge generation function and charge transport function simultaneously, the photosensitive layer can be formed by applying a liquid application in which a charge generation compound having a charge generation function, a charge transport compound having a charge transport function and a binder resin are dispersed or dissolved in a suitable solvent to an electroconductive substrate followed by optional drying. In addition, a plasticizing agent and/or a leveling agent can be added. With regard to the dispersion method of a charge generation material, the charge generation compound (material), the charge transport compound (material), the plasticizing agent, and the leveling agent, the same as mentioned above for the charge generation layer and the charge transport layer can be used. With regard to the binder resin, in addition to the binder resins mentioned for the charge transport layer, the binder resin for use in the charge generation layer can be mixed in combination. In addition, the charge transport polymers mentioned above can be also used. This is useful in light that mingling of the photosensitive layer compositions to the crosslinked surface layer can be reduced. The content of the charge generation material contained in the photosensitive layer is preferably from 10 to 30 %, the content of the binder resin contained therein is suitably from 20 to 80 % by weight and the charge transport compound is suitably from 10 to 70 parts by weight based on the total weight of the photosensitive layer.

The layer thickness of the photosensitive layer is suitably from about from about 5 to about 30 µm and preferably from about 10 to about 25 µm.

When the crosslinked surface layer is the surface portion of the photosensitive layer having a single layer structure, as described above, the crosslinked surface layer is formed by applying a liquid application for the crosslinked surface layer to the photosensitive layer followed by optional drying and thereafter curing the liquid upon irradiation of energy of light, heat or radiation. The layer thickness of the crosslinked surface layer is from 1 to 15 µm and preferably from 2 to 13 µm. When the layer thickness is too thick, cracking and layer peeling tend to occur. In addition, deterioration of the sensitivity tends to be significant. When the layer thickness is too thin, the durability is easily affected depending on the thickness.

### Single Layered Photosensitive Layer Formed of Charge Generation Layer Having Charge Generation Function

When the photosensitive layer is a charge generation layer having the charge generation function, the same charge generation layer as described for the photosensitive layer having a laminate structure can be used. The crosslinked surface layer provided on the charge generation layer contains a charge transport compound to have a charge transport function. The charge transport compound can contain a radical polymerizable function group. A charge transport compound having a radical polymerizable function group is suitably used since the anti-abrasion property significantly ameliorates. As described in the method of manufacturing a crosslinked surface layer, the crosslinked surface layer having a charge transport function is formed by applying the liquid application for the crosslinked surface layer for use in the present invention to the charge generation layer followed by optional drying and starting the curing reaction upon irradiation of light, heat, or radiation. The layer thickness of the crosslinked surface layer is from 10 to 30 µm and preferably from 10 to 25 µm. When the layer thickness is excessively thin, a sufficient charge voltage tends to be difficult to maintain. When the crosslinked surface layer is too thick, the crosslinked surface layer tends to be detached from the underlayer due to the volume constriction during curing.

### Intermediate Layer

In the image bearing member of the present invention, when the crosslinked surface layer forms the surface portion of the photosensitive layer, it is possible to provide an intermediate layer between the crosslinked surface layer and the photosensitive layer. This intermediate layer prevents inhibition of curing reaction and/or formation of a rough crosslinked surface layer caused by mingling of uncured photosensitive composition into the crosslinked surface layer containing a cured compound. Furthermore, it is possible to improve the adhesiveness between the photosensitive layer and the cross lining surface layer provided thereabove.

In the intermediate layer, a binder resin is used as a main component. Specific examples of the binder resins include, but are nor limited to, polyamide, alcohol soluble nylon, water soluble polyvinyl butyral, polyvinyl butyral and polyvinyl alcohol. Such an intermediate layer is formed by the typical method described above. The intermediate layer is suitably from 0.05 to 2 µm.

### Undercoating Layer

In the image bearing member of the present invention, an undercoating layer can be provided between an electroconductive substrate and a photosensitive layer. Such an undercoating layer is mainly made of a resin. Considering that a photosensitive layer is formed on such an undercoating layer (i.e., resin) using a solvent, the resin is preferably hardly soluble in a typically used organic solvent. Specific examples of such resins include, but not limited to, water soluble resins, for example, polyvinyl alcohol, casein, and sodium polyacrylate, alcohol soluble resins, for example, copolymerized nylon and methoxymethylized nylon and curing resins which form a three dimension mesh structure, for example, polyurethane, melamine resins, phenol resins, alkyd-melamine resins and epoxy resins. In addition, to prevent moiré and reduce the residual voltage, it is possible to add to an undercoating layer fine powder pigments of metal oxide, for example, titanium oxides, silica, alumina, zirconium oxides, tin oxides and indium oxides.

These undercoating layers can be formed by using a suitable solvent and a suitable coating method as described for the photosensitive layer. Silane coupling agents, titanium coupling agents and chromium coupling agents can be used in for the undercoating layer. Furthermore, an undercoating layer can be formed by using a material formed by anodizing Al₂O₃, or an organic compound, for example, polyparaxylylene (parylene) or an inorganic compound, for example, SiO₂, SnO₂, TiO₂, ITO, and CeO₂ by a vacuum thin-film forming method.

The layer thickness of such an undercoating layer is suitably from 0 to 5 µm.

### Addition of Anti-oxidizing Agent

Furthermore, in the present invention, to improve the environment resistance, in particular, to prevent the degradation of sensitivity and the rise in residual potential, an anti-oxidizing agent can be added to layers, i.e., the cross-linked surface layer, the charge generating layer, the charge transport layer, the undercoating layer and the adhesive layer.

Specific examples of the anti-oxidizing agent include, but not limited to, phenol compounds, paraphenylene diamines, hydroquinones, organic sulfur compounds, and organic phosphorous compounds.

### Phenol compounds

Specific examples of the phenol compound include, but not limited to, 2,6-di-t-butyl-p-cresol, butylated hydroxyanisol, 2,6-di-t-butyl-4-ethylphenol, stearyl-β-(3,5-di-t-butyl-4-hydroxyphenyl)propionate, 2,2'-methylene-bis-(4-methyl-6-t-butylphenol), 2,2'-methylene-bis-(4-ethyl-6-t-butylphenol), 4, 4'-thiobis-(3-methyl-6-t-butylphenol), 4,4'-butylidenebis-(3-methyl-6-t-butylphenol), 1,1,3-tris-(2-methyl-4-hydroxy-5-t-butylphenyl)butane, 1,3,5-trimethyl-2,4,6-tris(3,5-di-t-butyl-4-hydroxybenzyl)benzene, tetrakis-[methylene-3-(3',5'-di-t-butyl-4'-hydroxyphenyl)propionate]methane, bis[3,3'-bis(4'-hydroxy-3'-t-butylphenyl)butyric acid] glycol ester, and tocopherols.

### Paraphenylene Diamines

Specific examples of the paraphenylene diamines include, but not limited to, N-phenyl-N'-isopropyl-p-phenylenediamine, N,N'-di-sec-butyl-p-phenylenediamine, N-phenyl-N-sec-butyl-p-phenylenediamine, N,N'-di-isopropyl-p-phenylenediamine, and N,N'-dimethyl-N,N'-di-t-butyl-p-phenylenediamine.

### Hydroquinones

Specific examples of the hydroquinones include, but not limited to, 2,5-di-t-octylhydroquinone, 2,6-didodecylhydroquinone, 2-dodecylhydroquinone, 2-dodecyl-5-chlorohydroquinone, 2-t-octyl-5-methylhydroquinone, and 2-(2-octadecenyl)-5-methylhydroquinone.

### Organic Sulfur Compound

Specific examples of the organic sulfur compounds include, but not limited to, dilauryl-3,3'-thiodipropionate, distearyl-3,3'-thiodipropyonate, dimyristyl-3,3'-thiodipropionate, ditetradecyl-3,3'-thiodipropyonate and pentaerythritol tetrakis (3-laurylthio propionate).

### Organic Phosphorous Compound

Specific examples of organic phosphorous compounds include, but are not limited to, triphenyl phosphate, tris(nonylphenyl)phosphite, tri(dinonylphenyl)phosphite, tris(2-ethylhexyl)phosphite, tridecylphosphite, tris(tridecyl)phosphite, dinphenyl mono(2-ethylhexyl)phosphite, diphenyl monodecyl phosphite, tris(2,4,di-t-butylphenyl) phosphite, distearylpentaerythritol diphosphite, bis(2,4,di-t-butylphenyl)pentaerythritol phosphite, 2,2-methylenebis(4,6-di-t-butylphenyl)octyl phosphite, tetrakis(2,4-di-t-butylphenyl)4,4'-biphenylene-di-phosphite, dilaurylhydrogen phosphite, diphenyl hydrogen phosphite, tetraphenyl dipropylene glycol diphosphite, tetraphenyltetra(tridecyl)pentaerythritol tetraphosphite, tetra(tridecyl)-4,4' isopropylidene diphenyl diphosphite, bis(nonylphenyl)pentaerythritol diphosphite, and hydrogenerated bisphenol A pentaerythritol phosphite polymers.

These compounds are known as anti-oxidants for rubber, plastic, oils and products thereof are easily available in the market.

### Image Formation Method and Device

Next, the image formation method and the image forming apparatus of the present invention are described in detail with reference to the accompanying drawings.

The image forming method and the image forming apparatus of the present invention use the image bearing member having an uncured photosensitive layer and a cross linking surface layer containing a particular cured material and perform image forming processes of, for example, charging the image bearing member, irradiating the image bearing member to form a latent electrostatic image thereon, developing the latent electrostatic image with toner, transferring the toner image to an image bearing body (transfer medium), fixing the image and cleaning the surface of the image bearing member. The method in which a latent electrostatic image is directly transferred to a transfer medium followed by the development thereof does not necessarily have the processes mentioned above relating to the image bearing member.

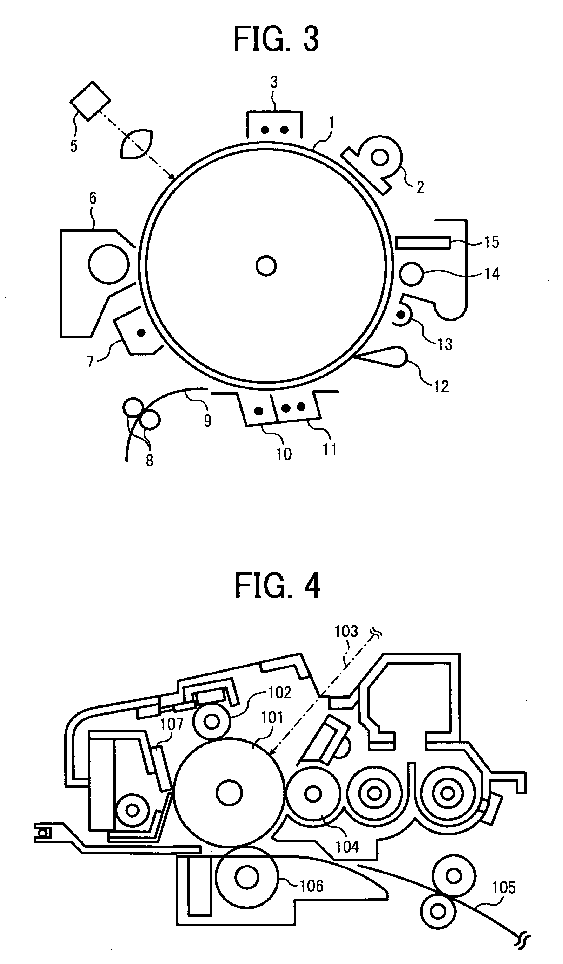

Fig. 3 is a schematic diagram illustrating an example of the image bearing member. A charging device 3 is used as the charging device to uniformly charge the image bearing member. Also, known charging devices, for example, a corotron device, a scorotron device, a solid discharging element, a needle electrode device, a roller charging device and an electroconductive brush device, can be used.

Next, an image irradiation portion 5 is used to form a latent electrostatic image on an image bearing member 1 which is uniformly charged. As the light source, typical luminescent materials, for example, a fluorescent lamp, a tungsten lamp, a halogen lamp, a mercury lamp, a sodium lamp, a luminescent diode (LED) , a semi-conductor laser (LD) and electroluminescence (EL) can be used. Various kinds of filters, for example, a sharp cut filter, a band pass filter, an infrared cut filter, a dichroic filter, a coherency filter and a color conversion filter can be used to irradiate the image bearing member 1 with light having only a desired wavelength.

Next, to visualize a latent electrostatic image formed on the image bearing member 1, a developing unit 6 is used. As the developing method, there are a single component development method and a two component development method which use a dry toner and a wet development method which uses a wet toner. When the image bearing member is positively (negatively) charged and image irradiation is performed, a positive (negative) latent electrostatic image is formed on the surface of the image bearing member 1. When this positive (negative) latent electrostatic image is developed with a toner (electric detecting particulates) having a negative (positive) polarity, a positive image is obtained. When the image is developed with a toner having a positive (negative) polarity, a negative image is obtained.

Next, a transfer charging device 9 is used to transfer the visualized toner image on the image bearing member 1 to a transfer medium 9. In addition, to perform a good transferring, a charging device 7 prior to transfer can be used. As a transfer device, an electrostatic transfer system using a transfer charging device or a bias roller, a mechanical transfer system using an adhesive transfer method or a pressure transfer method, and a magnetic transfer system can be used. As the electrostatic transfer system, the same device as the charging device mentioned above can be used.

Next, as a device to separate the transfer medium 9 from the image bearing member 1, a separation charging device 11 and a separation claw 12 are used. As other separating devices, electrostatic absorption guiding separation, side end belt separation, front end grip transfer, curvature separation, etc. can be used. As the separation charging device 11, the same device as the charging device mentioned above can be used.

Next, after transfer, to remove the toner remaining on the image bearing member 1, a fur brush 14 and a cleaning blade 15 are used. In addition, to effectively perform cleaning, a charging device 13 prior to cleaning can be used. Other cleaning devices, for example, a web-system device and a magnet brush system device can be also used. These cleaning devices can be used alone or in combination.

Next, if desired, a discharging device is used to remove the latent electrostatic image on the image bearing member 1. A discharging lamp 2 and a discharging charger can be used as the discharging device. The same devices as the irradiation light sources and the charging devices can be used therefor.

In addition to those mentioned above, known devices can be used in the processes of scanning originals, paper feeding, fixing images, discharging recording media, etc., which are performed not in the vicinity of the image bearing member 1.

The image forming method and the image forming apparatus of the present invention use the image bearing member of the present invention in the image formation device described above.

This image formation device can be fixedly implemented in a photocopier, a facsimile machine or a printer and also detachably incorporated therein as a form of a process cartridge. Fig. 4 is a diagram illustrating an example of the process cartridge.

A process cartridge for use in the image formation is a device (part) which includes an image bearing member 101 and at least one of a charging device 102, an irradiation device 103, a development device 104, a transfer device 106, a cleaning device 107 and a discharging device (not shown) and detachably attached to the main body of an image forming apparatus.

The image formation process by the process cartridge illustrated in Fig. 4 is: the image bearing member 101 in rotation is charged with the charging device 52 and irradiated by an irradiating device 53 to form a latent electrostatic image corresponding to the irradiation image on the surface of the image bearing member 101; the latent electrostatic image is developed with toner by the developing device 104; the toner image is transferred to a transfer medium 105 with the transfer device 106; the transferred image is then printed out; On the other hand, the surface of the image bearing member 101 is cleaned after transfer by the cleaning device 57 and discharged by the discharging device (not shown) ; and all the operations mentioned above continues in a repeated manner.

According to the present invention, there is provided a process cartridge for use in image formation which integrally has the image bearing member of the present invention having an uncured photosensitive layer and a cross linking surface layer containing a particular cured material and at least one of a charging device, a developing device, a transfer device, a cleaning device and a discharging device.

As apparent from the description above, the image bearing member of the present invention can be applied not only to an a photocopier using the electrophotographic system, but also widely to the applied electrophotographic field including, for example, a laser beam printer, a CRT printer, an LED printer, a liquid crystal printer, and laser plate making.

### Synthesis of Radical Reactive Compound Represented by Chemical Formula (B)

### (1) Synthesis of Example B1-1

Bromination reaction of triphenyl amine derivative is described.

Triphenyl amine derivatives are synthesized by a known method as raw material. For example, Ullman reaction between an iodobenzene derivative and aniline, Ullman reaction between a diphenyl amine derivative and an iodobenzene derivative and a coupling reaction between a diphenayl amine derivative and bromobenzene derivative under the presence of palladium catalyst can be suitably used.

Below is described a synthesis example of 4,4'-dimethyltriphenyl amine.

### Preparation of 4,4'-dimethyltriphenyl Amine

The following recipe is placed in a reaction container equipped with a stirrer, a thermometer, and a condenser and reacted in a nitrogen atmosphere at 210 °C for 25 hours. Subsequent to cooling, the resultant liquid is diluted with 200 ml of cyclohexane followed by absorption treatment with activated white earth and silica gel. 29.82 g of the end crystal (melting point: 109 °C) is obtained after condensation:

| | |
|---|---|
| Aniline (manufactured by Tokyo Chemical Industry Co., Ltd.) | 18. 63 g |
| p-iodotoluene | 89.39 g |
| potassium carbonate | 66.34 g |
| Copper powder | 1.27 g |

The thus obtained triphenyl amine derivative (a structure in which para position of at least one benzene ring is non-substituted) is brominated by a known bromination method. For example, a method using bromine or a method using N-bromosuccinimide (NBS) test reagent is suitable used.

A synthesis example about bromination 4,4'-dimethyl triphenyl amine is described below.

### Preparation of 4-bromo-4',4"-dimethyl Triphenyl Amine

14.53 g of 4,4'-dimethyl triphenyl amine and 30 ml of dioxane are placed in a reaction container equipped with a stirrer, a thermometer, and a dripping funnel and a liquid prepared by slowly dripping 9.34 g of bromine to 50 ml of dioxane at room temperature is slowly dripped thereto in a nitrogen atmosphere at room temperature followed by one hour reaction at the same temperature. Thereafter, 50 ml of toluene and 100 ml of water are added and the resultant organic layer is retrieved.

Subsequent to absorption treatment with sodium hydrogen carbonate and water and condensation, the end crystal is obtained (Yield amount: 15.78 g; Melting point: 102.0 to 103.0 °C).

### (2) Synthesis Example of B1-2

Boronation of 4-bromotriphenyl amine derivative is described below.

A boronation body of bromine group is synthesized by a known method. For example, a method in which a bromine body is treated by butyl lithium and reacted with trialcoxy boran or a method in which a bromine body is subjected to grignard reagent treatment and reacted with trialcoxy boran is suitably used.

A synthesis example of boronation of 4-bromo-4',4"-dimethyl triphenyl amine is described below.

### Preparation of 4-p-ditrilaminophenyl Boronic Acid

15.00 g of 4-bromo-4', 4"-dimethyl triphenyl amine and 100 ml of dehydrated tetrahydrofuran are placed in a reaction container equipped with a stirrer, a thermometer and a dripping funnel and cooled down to -76 °C in a nitrogen atmosphere. 25 ml of 6M butyl lithium hexane solution is slowly dripped to the reaction container and a solution of 13.32 g of trimethoxy boran and 5 ml of dehydrated tetrahydrofuran is slowly dripped. The resultant is stirred for 3 hours while still cooled and back to room temperature. 100 ml of 3N hydrochloric acid is added, hydrolyzed for 2 hours at room temperature, extracted by ethyl acetate and the obtained organic layer is water washed. Thereafter, the solvent is removed and the precipitated crystal is washed with hexane to obtain a coarse product. After recrystallization with a solvent mixture of cyclohexane and toluene, the end product (yield amount: 6.27 g, Melting point: 250 °C) is obtained.

### (3) Synthesis Example of B1-3

Synthesis of a biphenyl derivative by Suzuki-Miura coupling reaction between diphenyl aminophenyl boronate derivative and hydroxyl alkyl substituted bromobenzene derivative is described below.

Known reaction conditions are applicable in Suzuki-Miura coupling reaction. For example, a boronate derivative and a bromobenzene derivative are easily reacted by heating and stirring at 50 to 150 °C with a strong base and triphenyl phosphine palladium catalyst in a suitable solvent such as toluene and ethanol.

A synthesis examples of 2-(4'-di-p-tolylamino)(biphenyl-4-yl) ethyl alcohol is described below. Preparation of 2-(4'-di-p-tolylamino) (biphenyl-4-yl) ethyl alcohol

The following recipe is placed in a reaction container equipped with a stirrer, a thermometer, and a condenser and subjected to degassing treatment with ultrasonic wave in a nitrogen atmosphere. 1.05 g of tetrakis triphenyl phosphine palladium zero valent complex is added to the reaction container followed by reaction at 70 °C for 7 hours. Thereafter, the extracted organic layer is water washed and subject to absorption treatment twice with activated white earth followed by condensation to obtain a coarse product. The coarse product is refined with silica gel using column chromatography to obtain the end product (Yield amount: 15.75 g).

| | |
|---|---|
| 4-p-ditolylaminophenyl Boronic Acid | 14.51g |
| 2-(4-bromophenyl)ethyl alcohol (manufactured by Tokyo Kaseihin Co. Ltd.) | 9.22g |
| Toluene | 110 ml |
| 2M potassium carbonate aqueous solution | 110 ml |
| Ethanol | 55 ml |

### (4) Synthesis Example of B1-4 (Reactive compound represented by Chemical Formula (B))

(Meth)acryl reaction of an alcohol derivative is described.

Acrylization or methacrylization is conducted using an esterification reaction of acrylic acid or methacrylic acid and an alcohol derivative. In addition, an acrylic chloride or a methacrylic chloride can be used. In addition, with regard to acrylization, easy synthesis is possible by esterification of 3-chloropropanic acid chloride and an alcohol derivative followed by de-hydrochlorization under the presence of a base.

A synthesis example of the illustrated compound No. 3 {2-(4'(di-p-tolylamino)biphenyl-4-yl)ethyl acrylate is described below.

### Preparation of {2-(4'(di-p-tolylamino)biphenyl-4-yl)ethyl Acrylate

2 g of {2-(4'(di-p-tolylamino)biphenyl-4-yl)ethyl alcohol is dissolved in 18 ml of tetrahydrofuran and 0.771 g of triethyl amine is added in a nitrogen atmosphere. This solution is cooled down to 6 °C and 0.69 g of acrylic chloride is slowly dripped. Thereafter, the reaction is complete after a 5 hour stirring at room temperature. This reaction liquid is poured to water and extracted with toluene. The extracted liquid is water washed repeatedly. Thereafter, the solvent is removed from this toluene solution followed by refinement of column chromatography treatment (absorption medium: silica gel, developing solvent: n-hexane/ethyl acetate (1/2)). N-hexane is added to the obtained transparent oil to precipitate crystal. Thus, an acrylic ester derivative (transparent, yield amount: 1.89 g, melting point: 95 to 96 °C) of the illustrated compound No. 3 is obtained. The resultant is ionized by an atmospheric pressure chemical ionization method. The m/z value measured in the positive mode is 448 and matches the value obtained by adding 1 of proton to the molecular weight calculated by the mocleuclar formula.

It is possible to easily synthesize other illustrated compounds in the same manner as described above by changing the triphenyl amine derivative and the hydroxyl alkyl substituted-4-bromobenzene derivative in B1-1. In addition, when phenyl groups substituting N atoms in the anilines are different from each other, the illustrated compound is easily synthesized by the following steps: N-mono substitution is synthesized first; and then, the secondary amino group of the N-mono substitution is substituted with the other target phenyl group. In the first synthesis step of the N-mono substitution, for example, raw material aniline is acylized (and deacylized after the synthesis of the first N-mono substitution) to avoid production of primary amines, secondary amines, and tertiary amines simultaneously or secondary amines are selectively obtained by refinement (chromatography separation, separation by solubility difference among each salt of amine hydrochlorate to boiled water, etc.).

For example, the illustrated compound No. 14 is synthesized by preparing a synthesized product (2,4,6-trimethyl-2',4'-dimethyltriphenyl amine), which is prepared by cross coupling reaction between 2, 4-dimethyl diphenyl amine (Tokyo Chemical industry Co., Ltd.) and mesityl bromide (Tokyo Chemical industry Co., Ltd.) using an organic palladium catalyst, and using the same manner as described for the illustrated compound No. 3 for the processes after B1-1 process. The detail is described in the synthesis of the illustrated compound No. 14 described below.

The illustrated compound No. 26 is synthesized in the same manner as in the synthesis method for the illustrated compound No. 3 except that 2- (4-bromophenyl) ethyl alcohol and tetrakis triphenyl phosphine palladium zero valent complex for the illustrated compound No. 3 are replaced with 3- (4-chlorophenyl)propane-1-ol (manufactured by Acros Organic) and bis(tri-tert-butyl phosphine) palladium (0) (Strem Chemicals Inc.) and sodium tert butoxido and orthoxylene are used as the base and the solvent in Suzuki-Miura reaction. This can be also synthesized by using 3-(4-iodophenyl)-1-propanol, which can be prepared by iodinizing 3-phenyl-1-propanol under a suitable condition, instead of the hydroxyalkyl substituted bromobenzene derivative. The detail is described in the synthesis of the illustrated compound No. 26 described below. As described above, Suzuki-Miura coupling reaction is known to be conducted by an iodobenzene derivative and a phenyl boronic acid derivative. In B1-3 process, the hydroxyalkyl substituted bromobenzene derivative can be replaced with a hydroxyalkyl substituted iodobenzene derivative.

Furthermore, the illustrated compound No. 31 is synthesized by synthesizing 2,4, -dimethyl-4' -methyl triphenyl amine according to cross coupling reaction of 2,4-dimethyl diphenyl amine (manufactured by Tokyo Chemical Industry Co., Ltd.) and 4-bromotoluene (manufactured by Tokyo Chemical Industry Co., Ltd.) with an organic palladium catalyst and acrylating a hydroxyl compound prepared by cross coupling reaction between the boronic acid synthesized as described for the illustrated compound No. 3 and 4-(4-hydroxybutyl)bromo benzene.

The illustrated compound No. 47 can be synthesized in the manner in which N-mono substitution is synthesized and refined with acylized aniline in an amount corresponding to the half amount of p-iodotoluene for use in the illustrated compound No. 3, p-iodomethoxybenzene is reacted with the refined N-mono substitution and 4-bromophenethyl alcohol for use in the synthesis of the illustrated compound No. 3 is changed to 3-(4-bromophenyl)-sec-butyl alcohol.

Detailed synthesis Examples of the illustrated compounds Nos. 1, 2, 8, 9, 10, 13, 14, 16, 26, 30 and 35.

### Synthesis Example of Illustrated Example No. 1

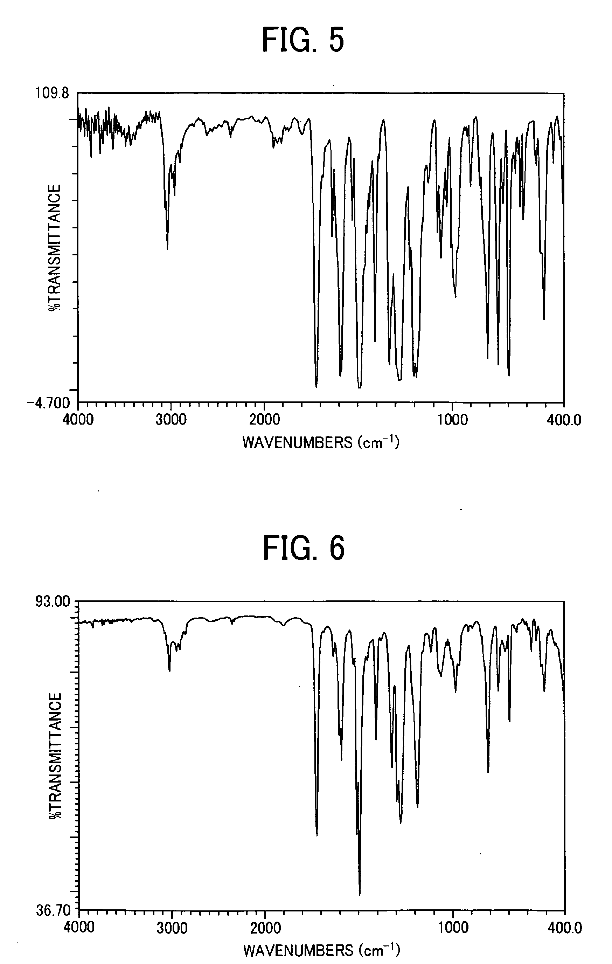

Insead of 4-p-ditolylaminophenyl boronic acid, 4-diphenylaminophenyl boronic acid is used to prepare 2-(4'-diphenylamino)biphenyl-4-yl)ethyl alcohol in the same manner as in synthesis of 2-(4'-(di-p-tolylamino)bihpneyl-4-il)ethyl alcohol. 8.1 g and 80 ml of N,N-dimethylacetamide are placed in a reaction container. Subsequent to stirring and dissolution, 4.22 g of 3-chloropropionyl chloride is dripped thereto in 15 minutes while cooled with ice. After cooling down to room temperature, the resultant is stirred for 150 minutes. Then, 12.4 ml of triethylamine is dripped thereto in 30 minutes followed by a 4 hour stirring at 60 °C. The liquid reaction is poured into 350 ml of water and hydrochloric acid is added thereto while strring in order that pH of the resultant is 5. After stirring is stopped and supernatant solution is removed, water is added followed by strring. This water wash treatment is repeated twice and the precipitation is extracted by ethyl acetate. The liquid extraction is furthermore washed with water three times. The solvent is removed by an evaporator to obtain 10 g of a coarse product. The coarse product is dissolved in 250 ml of toluene and 10 g of activated white earth (manufactured by Wako Pure Chemical Industries Ltd.) which has been dried at 130 °C beforehand is added thereto followed by a 30 minute stirring. The activated white earth is removed with filtration and the filtered resultant is condensed with an evaporator. The resultant is column fined using silica gel (60 N, manufactured by Merck Ltd.) with toluene as a developing solvent. The resultant is re-crystallized with a mixture solvent of ethanol and toluene to obtain 8.18 g of 2-(4'-(diphenylamino)biphenyl-4-yl)ethyl acrylate (target substance), which is non-color needle like crystal with the melting point of from 105.5 to 106.5 °C. The crystal is ionized by an atmospheric pressure chemical ionization method. The m/z value measured in the positive mode is 420 and matches the value obtained by adding 1 of proton to the molecular weight calculated by the molecular formula. The IR spectrum is measured and the result thereof is shown in Fig. 5.

### Synthesis Example of Illustrated Example No. 2

9.15 g of a coarse product of 2-(4'-(phenyl(p-tolyl)amino)biphenyl-4-yl)ethyl acrylate is obtained in the same manner as in Synthesis Example of Illustrated Example No. 1 except that 8.1 g of 2-(4'-(diphenylamino)biphenyl-4-yl)ethyl alcohol is replaced with 8.23 g of 2-(4'-(phenyl(p-tolyl)amino)biphenyl-4-yl-ethyl) alcohol prepared as in the synthesis of 2-(4'-di-p-tolylamino)biphenyl-4-yl)ethyl alcohol using 4-(phenyl(p-tolyl)amino)phenyl boronic acid.

The coarse product is dissolved in 250 ml of toluene and 10 g of activated white earth (manufactured by Wako Pure Chemical Industries Ltd.) which has been dried at 130 °C beforehand is added thereto followed by a 30 minute stirring. The activated white earth is removed with filtration and the filtered resultant is condensed with an evaporator. The resultant is column fined using silica gel (60 N, manufactured by Merck Ltd.) with toluene as a developing solvent. Thus, 13.3 g of the target substance of light yellow oil of 2-(4'-(phenyl(p-tolyl)amino)biphenyl-4-yl)ethyl acrylate. The oil is ionized by an atmospheric pressure chemical ionization method. The m/z value measured in the positive mode is 437 and matches the value obtained by adding 1 of proton to the molecular weight calculated by the molecular formula. The IR spectrum is measured and the result thereof is shown in Fig. 6.

### Synthesis of Illustrated Compound No. 8

The following recipe is placed in a reaction container and reacted at 100 °C for 7 hours in an argon gas atmosphere.

| | |
|---|---|
| 2,4-dimethyl diphenyl amine | 19.73 g |
| p-bromotoluene | 17.45 g |
| Sodium tert-butylate | 10.09g |
| Paradium (II) acetate | 0.112 g |
| Tri-tert-butyl phosphine | 0.8 ml |
| o-xylene | 600 ml |

The reaction solution is filtered using silica gel and the solvent is removed to obtain 39 g of a coarse product. The coarse product is column fined using silica gel to obtain 24.5 g of the target substance of non-color crystal of 2,4-dimethyl-4'-methyltriphenyl amine. The melting point thereof is from 76.5 to 78.0 °C.

23.8 g of 2,4-dimethyl-4'-methyltriphenyl amine is dissolved in 120 ml of chloroform and 15.48 g of N-bromosuccnine imide is slowly added thereto while cooled with ice followed by a 3 hour stirring at 12 °C. Subsequent to addition of 150 ml of water, the product is extracted with methylene chloride and the extracted solution is washed with water. Subsequent to dehydration with magnesium sulfonate, the solvent is removed to obtain 32 g of a coarse product. 100 ml of methanol is added thereto and washed while heated to obtain 31 g of a target substance (non-color crystal) of 4-bromo-2',4'-dimethyl-4"-methyltriphenyl amine. The melting point thereof is from 106.2 to 108.0 °C.

31 g of 4-bromo-2',4'-dimethyl-4"-methyltriphenyl amine and 200 ml of tetrahydrofuran are dissolved and cooled down to -74 °C in nitrogen atmosphere and 45 ml of 2.6 mol/l of n-butyl lithium hexane solution is slowlu dripped thereto. After a one hour stirring, 20 ml of trimethyl borate diluted with tetrahydrofuran is slowly dripped thereto followed by a three hour stirring. The resultant is back to room temperature and 100 ml of trinormal hydrochloric acid is slowly dripped followed by a one hour stirring at room temperature. The resultant is extracted with ethyl acetate and washed with water. Subsequent to removal of the solvent, 23.9 g of the target substance of 4-(2,4-dimethylphenyl(4-methylphenyl)amino)phenyl boronic acid is obtained.

The following recipe is placed in a reaction container and stirred while subject to ultrasonic wave for 20 minutes in argon gas atmosphere.

| | |
|---|---|
| 2-(4-bromophenyl)ethyl alcohol | 5.03 g |
| 4-(2,4-dimethylphenyl(4-methylphenyl)amino)phenyl boronic acid | 8.28 g |
| Toluene | 70 ml |
| Ethanol | 35 ml |
| 2M potassium carbonate aqueous solution | 70 ml |

Thereafter, 0.58 g of tetrakis(triphenylphosphine)palladium(0) is added thereto followed by a 7 hour stirring at 70 °C. After cooled down, the resultant is extracted with toluene and washed with water. Subsequent to removal of the solvent, 12 g of a coarse product is obtained. The coarse product is column fined using silica gel and thus 4.52 g of non-color amorphouns 2-(4'-(2,4-dimethylphenyl(4-methylphenyl)amino)biphenyl-4-yl)ethy 1 alcohol is obtained.

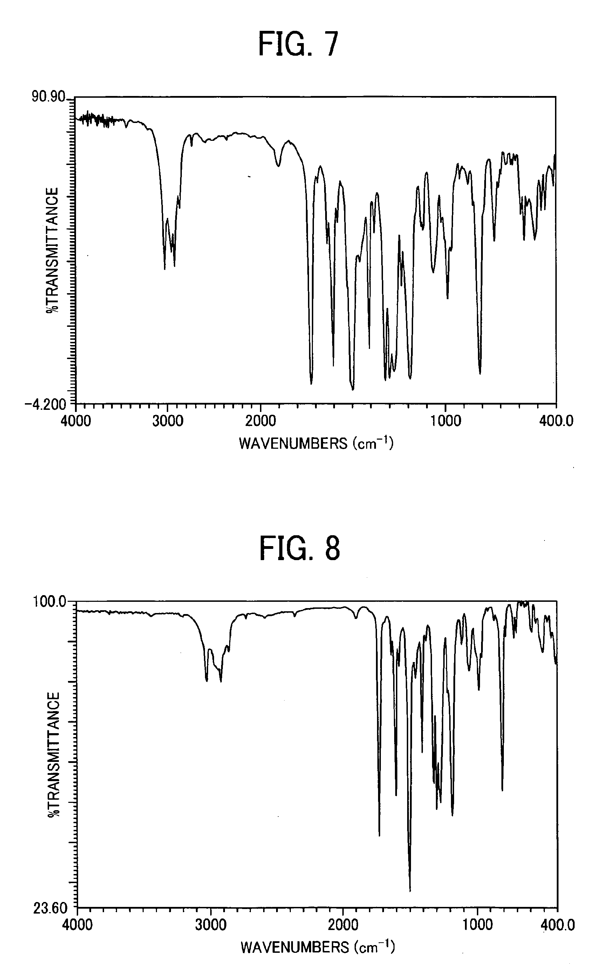

In a reaction container, 4.4 g of 2-(4'-(2,4-dimethylphenyl(4-methylphenyl)amino)biphenyl-4-yl)ethy 1 alcohol and 40 ml of N,N-dimethylacetamide are placed followed by stirring and dissolution. 2.06 g of 3-chloropropionyl chloride is dripped thereto in 15 minutes while cooled with ice. The resultant is back to room temperature and stirred for 120 minutes. Thereafter, 6.1 ml of triethylamine is dripped in 30 minutes followed by a 4 hour stirring at 60 °C. The reaction solution is poured into 350 ml of water and hydrochloric acid is added while stirred in order that pH thereof is 5. After stirring is stopped and supernatant solution is removed, water is added followed by strring. This water wash treatment is repeated twice and the precipitation is extracted by methylene chloride. The liquid extraction is furthermore washed with water three times. The solvent is removed by an evaporator to obtain 7 g of a coarse product. The coarse product is dissolved in 100 ml of toluene and 5 g of activated white earth (manufactured by Wako Pure Chemical Industries Ltd.) which has been dried at 130 °C beforehand is added thereto followed by a 30 minute stirring. The activated white earth is removed with filtration and the filtered resultant is condensed with an evaporator. The resultant is column fined using silica gel (60 N, manufactured by Merck Ltd.) with toluene as a developing solvent and thus, 4.46 g of the target substance (non-color oil) of 2-(4'-(2,4-dimethylphenyl(4-methylphenyl)amino)biphenyl-4-yl)ethy 1 acrylate is obtained. The oil is ionized by an atmospheric pressure chemical ionization method. The m/z value measured in the positive mode is 462 and matches the value obtained by adding 1 of proton to the molecular weight calculated by the molecular formula. The IR spectrum is measured and the result thereof is shown in Fig. 7.

### Synthesis of Illustrated Compound No. 9

3,4-dimethyl-4'-methyltriphenyl amine is synthesized from 4-methyldiphenyl amine and 4-bromo-o-xylene in the same manner as in 2,4-dimethyl-4'methyltriphenyl amine. 13.78 g of 4-bromo-3',4'-dimethyl-4"-methyltriphenyl amine synthesized from 3,4-dimethyl-4'-methyltriphenyl amine as in synthesis of 4-bromo-2',4'-dimethyl-4"-methyltriphenyl amine is dissolved in 100 ml of tetrahydrofuran and cooled down to -76 °C in nitrogen atmosphere. Then, 17.5 ml of 2.6 mol/l of n-butyl lithium hexane solution is slowly dripped thereto. Thereafter, the resultant is slowly stirred for one hour. A solution in which 5.88 g of trimethylborate is diluted with 3 g of tetrahydrofuran is slowly dripped to the resultant. Subsequent to a 4 hour stirring, the temperature of the resultant is back to room temperature and 84 ml of mono-normal hydrochloric acid is slowly drippepd followed by a one hour stirring at room temperature. The resultant is extracted with ethyl acetate and washed with water. The solvent is removed and washed with hexane and 6.87 g of the target substance of 4-(3,4-dimethylphenyl(4-methylphenyl)amino)phenyl boronic acid is obtained.

The following recipe is placed in a reaction container and stirred while subject to ultrasonic wave for 20 minutes in argon gas atmosphere.

| | |
|---|---|
| 2-(4-bromophenyl)ethyl alcohol | 4.19 g |
| 4-(3,4-dimethylphenyl(4-methylphenyl)amino)phenyl boronic acid | 6.87 g |
| Toluene | 36 g |
| Ethanol | 16.4 g |
| 2M potassium carbonate aqueous solution | 53 g |

Thereafter, 24 mg of tetrakis(triphenylphosphine)palladium(0) is added thereto followed by a 3 hour stirring at 60 °C. After cooled down, the resultant is extracted with toluene and washed with water. Subsequent to removal of the solvent, 9.32 g of a coarse product is obtained. The coarse product is column fined using silica gel and thus 6.12 g of non-color oil 2-(4'-(3,4-dimethylphenyl(4-methylphenyl)amino)biphenyl-4-yl) ethyl alcohol is obtained.

5.49 g of 2-(4'-(3,4-dimethylphenyl(4-methylphenyl)amino)biphenyl-4-yl) ethyl alcohol and 43 g of N,N-dimethylacetamide are placed in a reaction container, followed by stirring and dissolution. 2.1 g of 3-chloropropionyl chloride is dripped thereto in 10 minutes while cooled with ice. The resultant is back to room temperature and stirred for 30 minutes. Thereafter, 6.6 g of triethylamine is dripped in 30 minutes followed by a 4 hour stirring at 60 °C. The reaction solution is poured into 100 ml of water and the product is extractd with ethyl acetate. The liquid extraction is washed with saturated saline three times and dehydrated with sodium sulfonate. The solvent is removed by an evaporator to obtain 6.87 g of a coarse product. The coarse product is column fined using silica gel (60 N, manufactured by Merck Ltd.) with toluene as a developing solvent and thus, 5.5 g of the target substance (non-color oil) of 2-(4'-(3,4-dimethylphenyl(4-methylphenyl)amino)biphenyl-4-yl) ethyl acrylate is obtained. The oil is ionized by an atmospheric pressure chemical ionization method. The m/z value measured in the positive mode is 462 and matches the value obtained by adding 1 of proton to the molecular weight calculated by the molecular formula. The IR spectrum is measured and the result thereof is shown in Fig. 8.

### Synthesis of Illustrated Compound No. 10

2,4,6-trimethyl-4'-methyltriphenyl amine is synthesized from 4-methyldiphenyl amine and mesityl bromide in the same manner as in synthesis of 2,4-dimethyl-4'methyltriphenyl amine. 17.58 g of 4-bromo-2',4',6'-trimethyl-4"-methyltriphenyl amine synthesized from 2,4,6-trimethyl-4'-methyltriphenyl amine as in synthesis of 4-bromo-2',4'-dimethyl-4"-methyltriphenyl amine is dissolved in 150 ml of tetrahydrofuran and cooled down to -75 °C in nitrogen atmosphere. Then, 22 ml of 2.6 mol/l of n-butyl lithium hexane solution is slowly dripped thereto. Thereafter, the resultant is stirred for one hour. A solution in which 9.61 g of trimethylborate is diluted with 10 ml of tetrahydrofuran is slowly dripped to the resultant. Subsequent to a 4 hour stirring, the temperature of the resultant is back to room temperature and 108 ml of mono-normal hydrochloric acid is slowly drippepd followed by a one hour stirring at room temperature. The resultant is extracted with ethyl acetate and washed with water. The solvent is removed and 15.15 g of the target substance of 4-(2,4,6-trimethylphenyl(4-methylphenyl)amino)phenyl boronic acid is obtained.

The following recipe is placed in a reaction container and stirred while subject to ultrasonic wave for 20 minutes in argon gas atmosphere.

| | |
|---|---|
| 2-(4-bromophenyl)ethyl alcohol | 9.7 g |
| 4-(2,4,6-trimethylphenyl(4-methylphenyl)amino)phenyl boronic acid | 15.15g |
| Toluene | 66 ml |
| Ethanol | 33 ml |
| 2M potassium carbonate aqueous solution | 84 g |

Thereafter, 0.51 g of tetrakis(triphenylphosphine)palladium(0) is added thereto followed by a 4 hour stirring at 60 °C. After cooled down, the resultant is extracted with toluene and washed with water. Subsequent to removal of the solvent, 21.85 g of a coarse product is obtained. The coarse product is column fined using silica gel and thus 11.36 g of non-color oil 2-(4'-(2,4,6-trimethylphenyl(4-methylphenyl)amino)biphenyl-4-yl) ethyl alcohol is obtained.

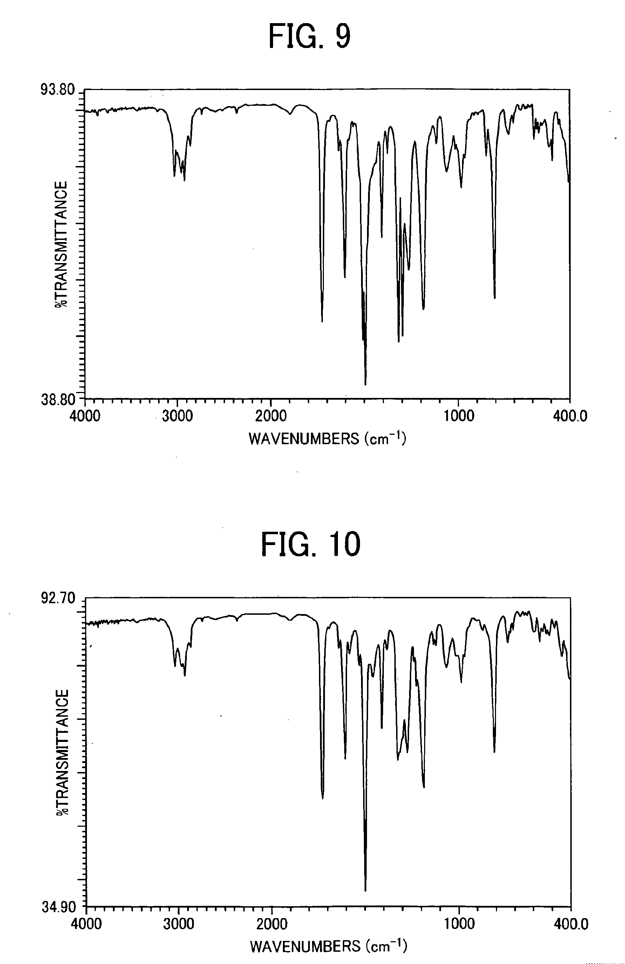

11.36 g of 2-(4'-(2,4,6-trimethylphenyl(4-methylphenyl)amino)biphenyl-4-yl) ethyl alcohol and 80 ml of N,N-dimethylacetamide are placed in a reaction container, followed by stirring and dissolution. 5.13 g of 3-chloropropionyl chloride is dripped thereto in 15 minutes while cooled with ice. The temperature of the resultant is made back to room temperature and stirred for 150 minutes. Thereafter, 15 ml of triethylamine is dripped in 30 minutes followed by a 4 hour stirring at 60 °C. The reaction solution is poured into 500 ml of water and hydrochloric acid is added while stirred in order that pH thereof is 4. The reaction product is extracted by 300 ml of ethyl acetate and then washed with water three times. The solvent is removed by an evaporator to obtain 14 g of a coarse product. The coarse product is dissolved in 300 ml of toluene and 10 g of activated white earth (manufactured by Wako Pure Chemical Industries Ltd.) which has been dried at 130 °C beforehand is added thereto followed by a 30 minute stirring. The activated white earth is removed with filtration and the filtered resultant is condensed with an evaporator. The resultant is column fined using silica gel (60 N, manufactured by Merck Ltd.) with toluene as a developing solvent and thus, 10.5 g of the target substance (non-color oil) of 2-(4'-(2,4,6-trimethylphenyl(4-methylphenyl)amino)biphenyl-4-yl) ethyl acrylate is obtained. The oil is ionized by an atmospheric pressure chemical ionization method. The m/z value measured in the positive mode is 476 and matches the value obtained by adding 1 of proton to the molecular weight calculated by the molecular formula. The IR spectrum is measured and the result thereof is shown in Fig. 9.

### Synthesis of Illustrated Compound No. 13

2,4-dimethyl-3',4'-dimethyltriphenyl amine is synthesized from 2,4-dimethyldiphenyl amine and 4-bromo-o-xylene in the same manner as in 2,4-dimethyl-4'methyltriphenyl amine. 15.4 g of 4-bromo-2',4'-dimethyl-3",4"-dimethyltriphenyl amine synthesized from 2,4-dimethyl-3',4'-dimethyltriphenyl amine as in synthesis of 4-bromo-2',4'-dimethyl-4"-methyltriphenyl amine is dissolved in 100 ml of tetrahydrofuran and cooled down to -75 °C in nitrogen atmosphere. Then, 19 ml of 2.6 mol/l of n-butyl lithium hexane solution is slowly dripped thereto. Thereafter, the resultant is stirred for one hour. A solution in which 6.32 g of trimethylborate is diluted with 5 g of tetrahydrofuran is slowly dripped to the resultant. Subsequent to a 3 hour stirring, the temperature of the resultant is back to room temperature and 84 ml of mono-normal hydrochloric acid is slowly drippepd followed by a one hour stirring at room temperature. The resultant is extracted with ethyl acetate and washed with water. The solvent is removed and 14.46 g of the target substance of 4-(2,4-dimethylphenyl(3,4-dimethylphenyl)amino)phenyl boronic acid is obtained.

The following recipe is placed in a reaction container and stirred while subject to ultrasonic wave for 20 minutes in argon gas atmosphere.

| | |
|---|---|
| 2-(4-bromophenyl)ethyl alcohol | 9.3 g |
| 4-(2,4-dimethylphenyl(3,4-dimethylphenyl)amino)phenyl boronic acid | 13.35g |
| Toluene | 67.2 g |
| Ethanol | 30.5 g |
| 2M potassium carbonate aqueous solution | 98.7 g |

Thereafter, 0.224 g of tetrakis(triphenylphosphine)palladium(0) is added thereto followed by a 4 hour stirring at 60 °C. After cooled down, the resultant is extracted with toluene and washed with water. Subsequent to removal of the solvent, 21.73 g of a coarse product is obtained. The coarse product is column fined using silica gel and thus 15.51 g of non-color oil 2-(4'-(2,4-dimethylphenyl(3,4-dimethylphenyl)amino)biphenyl-4-yl) ethyl alcohol is obtained.

8.67 g of 2-(4'-(2,4-dimethylphenyl(3,4-dimethylphenyl)amino)biphenyl-4-yl) ethyl alcohol and 72 ml of N,N-dimethylacetamide are placed in a reaction container, followed by stirring and dissolution. 3.1 g of 3-chloropropionyl chloride is dripped thereto in 15 minutes while cooled with ice. The temperature of the resultant is made back to room temperature and stirred for 120 minutes. Thereafter, 14 ml of triethylamine is dripped in 30 minutes followed by a 4 hour stirring at 60 °C. The reaction solution is poured into 200 ml of water and hydrochloric acid is added while stirred in order that pH thereof is 4. The reaction product is extracted by 300 ml of ethyl acetate and then washed with water three times. The solvent is removed by an evaporator to obtain 11 g of a coarse product. The coarse product is dissolved in 200 ml of toluene and 9 g of activated white earth (manufactured by Wako Pure Chemical Industries Ltd.) which has been dried at 130 °C beforehand is added thereto followed by a 30 minute stirring. The activated white earth is removed with filtration and the filtered resultant is condensed with an evaporator. The resultant is column fined using silica gel (60 N, manufactured by Merck Ltd.) with toluene as a developing solvent and thus, 9.94 g of the target substance (light yellow colored oil) of 2-(4'-(2,4-dimethylphenyl(3,4-dimethylphenyl)amino)biphenyl-4-yl) ethyl acrylate is obtained. The oil is ionized by an atmospheric pressure chemical ionization method. The m/z value measured in the positive mode is 476 and matches the value obtained by adding 1 of proton to the molecular weight calculated by the molecular formula. The IR spectrum is measured and the result thereof is shown in Fig. 10.

### Synthesis of Illustrated Compound No. 14

2,4-dimethyl-2',4',6'-trimethyltriphenyl amine is synthesized from 2,4-dimethylphenyl amine and mesityl bromide in the same manner as in synthesis of 2,4-dimethyl-4'methyltriphenyl amine. 25.16 g of 4-bromo-2',4'-dimethyl-2",4",6"-trimethyltriphenyl amine (the melting point: 134.0 to 137.0 °C) synthesized from 2,4-dimethyl-2',4',6'-trimethyltriphenyl amine as in synthesis of 4-bromo-2',4'-dimethyl-4"-methyltriphenyl amine is dissolved in 100 ml of tetrahydrofuran and cooled down to -77 °C in nitrogen atmosphere. Then, 30 ml of 2.6 mol/l of n-butyl lithium hexane solution is slowly dripped thereto. Thereafter, the resultant is stirred for one hour. A solution in which 9.95 g of trimethylborate is diluted with 5 g of tetrahydrofuran is slowly dripped to the resultant. Subsequent to a 6 hour stirring, the temperature of the resultant is back to room temperature and 220 ml of mono-normal hydrochloric acid is slowly drippepd followed by a one hour stirring at room temperature. The resultant is extracted with ethyl acetate and washed with water. The solvent is removed and 23 g of the target substance of 4-(2,4-dimethylphenyl(2,4,6-trimethylphenyl)amino)phenyl boronic acid is obtained.

The following recipe is placed in a reaction container and stirred while subject to ultrasonic wave for 20 minutes in argon gas atmosphere.

| | |
|---|---|
| 2-(4-bromophenyl)ethyl alcohol 4-(2,4,-dimethylphenyl(2,4,6-trimethylphenyl)amino)phenyl | 15.5 g |
| boronic acid | 22.92 g |
| Toluene | 86.7 g |
| Ethanol | 39.5 g |
| 2M potassium carbonate aqueous solution | 128 g |

Thereafter, 0.37 g of tetrakis(triphenylphosphine)palladium(0) is added thereto followed by a 3 hour stirring at 60 °C. After cooled down, the resultant is extracted with toluene and washed with water. Subsequent to removal of the solvent, 35.29 g of a coarse product is obtained. The coarse product is column fined using silica gel and thus 30 g of light brown colored oil 2-(4'-(2,4-dimethylphenyl(2,4,6-trimethylphenyl)amino)biphenyl-4-yl) ethyl alcohol is obtained.

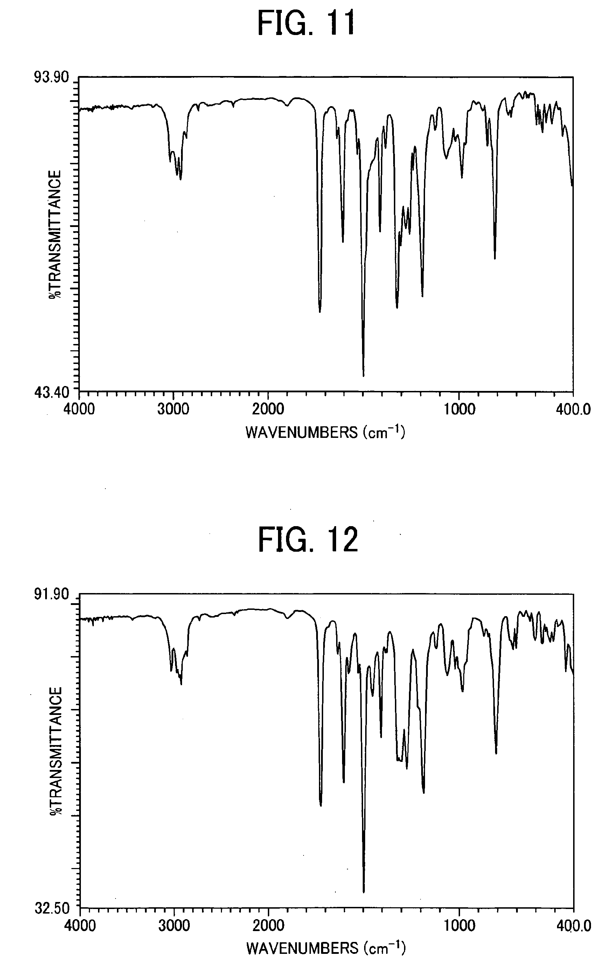

9.69 g of 2-(4'-(2,4-dimethylphenyl(2,4,6-trimethylphenyl)amino)biphenyl-4-yl) ethyl alcohol and 50 g of tetrahydrofuran are placed in a reaction container, followed by stirring and dissolution. 3.1 g of 3-chloropropionyl chloride is dripped thereto in 10 minutes while cooled with ice. Subsequent to a 30 minute stirring, 12.2 g of triethylamine is dripped in 40 minutes followed by an 8 hour stirring at 62 °C. The reaction solution is poured into 200 ml of water and hydrochloric acid is added while stirred in order that pH thereof is 4. The reaction product is extracted by 300 ml of ethyl acetate and then washed with water three times. The liquid extraction is furthermore washed with water three times. The solvent is removed by an evaporator to obtain 10.29 g of a coarse product. The coarse product is dissolved in 200 ml of toluene and 9 g of activated white earth (manufactured by Wako Pure Chemical Industries Ltd.) which has been dried at 130 °C beforehand is added thereto followed by a 30 minute stirring. The activated white earth is removed with filtration and the filtered resultant is condensed with an evaporator. The resultant is column fined using silica gel (60 N, manufactured by Merck Ltd.) with toluene as a developing solvent and thus, 9 g of the target substance (non-color oil) of 2-(4'-(2,4-dimethylphenyl(2,4,6-trimethylphenyl)amino)biphenyl-4-yl) ethyl acrylate is obtained. The oil is ionized by an atmospheric pressure chemical ionization method. The m/z value measured in the positive mode is 490 and matches the value obtained by adding 1 of proton to the molecular weight calculated by the molecular formula. The IR spectrum is measured and the result thereof is shown in Fig. 11.

3,4-dimethyl-3',4'-dimethyltriphenyl amine is synthesized from bis(3,4-dimethylphenyl)amine and bromobenzene as in the synthesis of 2,4-dimethyl-4'-methyl triphenyl amine. Bromination and boronization are conducted thereafter as in the synthesis of the illustrated compound No. 8 to synthesize 4-(bis(3,4-dimethylphenyl)amino)phenyl boronic acid. 8 g of non-color amorphous of 2- (4'-(bis(3,4-dimethylphenyl)amino)biphenyl-4-yl) ethyl alcohol is synthesized from 3,4-dimethyl-3',4'-dimethyltriphenyl amine and 2-(4-bormophenyl) ethyl alcohol as in the synthesis of 2-(4'-(2,4,6-trimethylphenyl(4-methylphenyl)amino)biphenyl-4-yl) ethyl alcohol of the illustrated compound No. 10.

7.78 g of 2-(4'- (bis (3, 4-dimethylphenyl) amino) biphenyl-4-yl) ethyl alcohol and 44 g of tetrahydrofuran are placed in a reaction container, followed by stirring and dissolution. 2.9 g of 3-chloropropionyl chloride is dripped thereto in 10 minutes while cooled with ice. The resultant is stirred for 30 minutes at room temperature. Thereafter, 9.2 g of triethylamine is dripped in 60 minutes followed by a 19 hour stirring at 60 °C. The reaction solution is poured into 350 ml of water and hydrochloric acid is added while stirred in order that pH thereof is 5. The reaction product is extracted by 300 ml of ethyl acetate and then washed with water three times. The solvent is removed by an evaporator to obtain 9.94 g of a coarse product. The coarse product is dissolved in 180 ml of toluene and 8 g of activated white earth (manufactured by Wako Pure Chemical Industries Ltd.) which has been dried at 130 °C beforehand is added thereto followed by a 30 minute stirring. The activated white earth is removed with filtration and the filtered resultant is condensed with an evaporator. The resultant is column fined using silica gel (60 N, manufactured by Merck Ltd.) with toluene as a developing solvent and thus, 7.88 g of the target substance (non-color crystal having a melting point of from 86.5 to 87.5 °C) of 2-(4'-(bis(3,4-dimethylphenyl)amino)biphenyl-4-yl) ethyl acrylate is obtained. The crystal is ionized by an atmospheric pressure chemical ionization method. The m/z value measured in the positive mode is 476 and matches the value obtained by adding 1 of proton to the molecular weight calculated by the molecular formula. The IR spectrum is measured and the result thereof is shown in Fig. 12.

### Synthesis of Illustrated Compound No. 26

37.5 g of 3-phenyl-1-propanol is dissolved in 200 ml of acetic acid and 0.8 ml of concentrated sulfuric acid is added to the solution followed by a 90 minute stirring at 65 °C. Thereafter, 31.46 g of iodine and 12.56 g of periodic acid are added thereto to conduct a reaction for 4 hours at 80 °C. The reaction liquid is poured into 600 ml of water and stirred. The supernatant solution is discarded and sodium sulfite aqueous solution is added followed by a one hour stirring. The oil layer is washed with water several times and diluted with methylene chloride and dehydrated with magnesium sulfate. The solvent is removed to obtain 81.12 g of the light yellow colored oily target substance. According to a chemical composition analysis by a high speed liquid chromatograph mass spectrometer, the substance is a mixture of 79 % of 3-(4-iodophenyl) propyl acetate and 21 % of 3-(2-iodophenyl) propyl acetate.

40 g of the mixture of 3-(4-iodophenyl) propyl acetate and 3-(2-iodophenyl)propyl acetate prepared above is dissolved in 150 ml of ethanol and an aqueous solution in which 10.52 g of sodium hydrates dissolved in 119 ml of water is added thereto. Subsequent to a 40 minute stirring at 65 °C, the reaction liquid is poured into 400 ml of water and 15 ml of concentrated hydrochloric acid is added for neutralization. The oil layer is washed with water several times and diluted with methylene chloride and dehydrated with magnesium sulfate. The solvent is removed to obtain 32.4 g of the light yellow colored oily target substance. According to a chemical composition analysis by a high speed liquid chromatograph mass spectrometer, the substance is a mixture of 78 % of 3(4-iodophenyl)-1-propanol and 22 % of 3-(2-iodophenyl)-1-propanol.

The following recipe is placed in a reaction container and stirred while subject to ultrasonic wave for 10 minutes in argon gas atmosphere.

| | |
|---|---|
| Mixture of 3-(4-iodophenyl)-1-propanol and 3-(2-iodophenyl)-1-propanol prepared above | 7.23 g |
| 4-(di-p-tolylamino)phenyl boronic acid | 7.0 g |
| Toluene | 70 ml |
| Ethanol | 35 ml |
| 2M potassium carbonate aqueous solution | 70 ml |

Thereafter, 0.51 g of tetrakis(triphenylphosphine)palladium(0) is added thereto followed by an 8 hour stirring at 70 °C. After cooled down, the resultant is extracted with toluene and washed with water. Subsequent to removal of the solvent, 10 g of a coarse product is obtained. The coarse product is column fined using silica gel and thus 5.09 g of non-color amorphous of 3-(4'-(di-p-tolylamino)biphenyl-4-yl)-1-propanol is obtained. According to a chemical composition analysis by a high speed liquid chromatograph mass spectrometer, the absorption peak area ratio at a wavelength of 254 nm is 99.8 % and no product derived from 3-(2-iodophenyl)-1-propanol is contained.

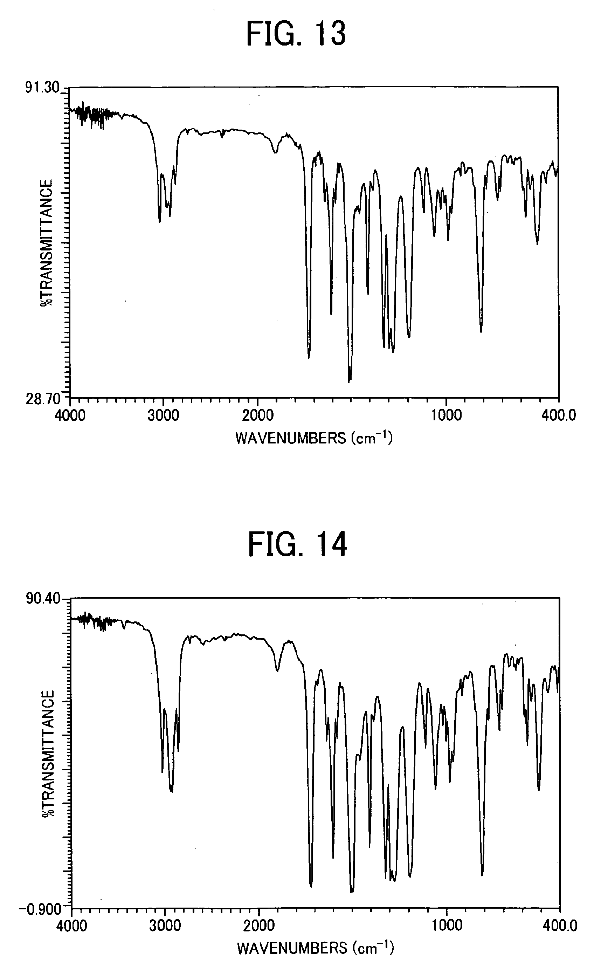

5.09 g of 3-(4'-(di-p-tolylamino)biphenyl-4-yl)-1-propanol and 50 ml of N,N-dimethylacetamide are placed in a reaction container, followed by stirring and dissolution. 2.38 g of 3-chloropropionyl chloride is dripped thereto in 15 minutes while cooled with ice. The temperature of the resultant is made back to room temperature and stirred for 180 minutes. Thereafter, 5.1 ml of triethylamine is dripped in 30 minutes followed by a 3 hour stirring at 60 °C. The reaction solution is poured into 350 ml of water and hydrochloric acid is added while stirred in order that pH thereof is 4. The reaction product is extracted by 300 ml of ethyl acetate and then washed with water three times. The solvent is removed by an evaporator to obtain 6.23 g of a coarse product. The coarse product is dissolved in 130 ml of toluene and 5 g of activated white earth (manufactured by Wako Pure Chemical Industries Ltd.) which has been dried at 130 °C beforehand is added thereto followed by a 30 minute stirring. The activated white earth is removed with filtration and the filtered resultant is condensed with an evaporator. The resultant is column fined using silica gel (60 N, manufactured by Merck Ltd.) with toluene as a developing solvent and thus, 5.65 g of the target substance (non-color oil) of 3-(4'-(di-p-tolylamino)biphenyl-4-yl) propyl acrylate is obtained. The oil is ionized by an atmospheric pressure chemical ionization method. The m/z value measured in the positive mode is 462 and matches the value obtained by adding 1 of proton to the molecular weight calculated by the molecular formula. The IR spectrum is measured and the result thereof is shown in Fig. 13.

### Synthesis of Illustrated Compound No. 30

49.8 g of 4-phenyl-1-butanol is dissolved in 270 ml of acetic acid and 1.1 ml of concentrated sulfuric acid is added to the solution followed by a 150 minute stirring at 65 °C. Thereafter, 38.01 g of iodine and 15.17 g of periodic acid are added thereto to conduct a reaction for 5 hours at 70 °C and 2 hours at 80 °C. The reaction liquid is poured into 800 ml of water and stirred. The supernatant solution is discarded and sodium sulfite aqueous solution is added followed by a 3 hour stirring. The oil layer is washed with water several times and diluted with methylene chloride and dehydrated with magnesium sulfate. The solvent is removed to obtain 106.16 g of the light yellow colored oily target substance. According to a chemical composition analysis by a high speed liquid chromatograph mass spectrometer, the substance is a mixture of 79 % of 4- (4-iodophenyl) butyl acetate and 21 % of 4-(2-iodophenyl) butyl acetate.

60 g of the mixture of 4-(4-iodophenyl) butyl acetate and 4- (2-iodophenyl) butyl acetate prepared above is dissolved in 225 ml of ethanol and an aqueous solution in which 15.12 g of sodium hydrates dissolved in 170 ml of water is added thereto. Subsequent to a 60 minute stirring at 65 °C, the reaction liquid is poured into 600 ml of water and 17.5 ml of concentrated hydrochloric acid is added for neutralization. The oil layer is washed with water several times and diluted with methylene chloride and dehydrated with magnesium sulfate. The solvent is removed to obtain 46.91 g of the light yellow colored oily target substance. According to a chemical composition analysis by a high speed liquid chromatograph mass spectrometer, the substance is a mixture of 79 % of 4-(4-iodophenyl)-1-butanol and 21 % of 4-(2-iodophenyl)-1-butanol.

The following recipe is placed in a reaction container and stirred while subject to ultrasonic wave for 10 minutes in argon gas atmosphere.

| | |
|---|---|
| Mixture of 4-(4-iodophenyl)-1-butanol and 4-(2-iodophenyl)-1-butanol prepared above | 8.24 g |
| 4-(di-p-tolylamino) phenyl boronic acid | 7.0 g |
| Toluene | 70 ml |
| Ethanol | 35 ml |
| 2M potassium carbonate aqueous solution | 70 ml |

Thereafter, 0.51 g of tetrakis(triphenylphosphine)palladium(0) is added thereto followed by an 8 hour stirring at 70 °C. After cooled down, the resultant is extracted with toluene and washed with water. Subsequent to removal of the solvent, 10 g of a coarse product is obtained. The coarse product is column fined using silica gel and thus 6.02 g of non-color oily substance of 4-(4'-(di-p-tolylamino)biphenyl-4-yl)-1-butanol is obtained. According to a chemical composition analysis by a high speed liquid chromatograph mass spectrometer, the absorption peak area ratio at a wavelength of 254 nm is 99.2 % and no product derived from 4-(2-iodophenyl)-1-butanol is contained.

6 g of 4- (4'- (di-p-tolylamino) biphenyl-4-yl) -1-butanol and 50 ml of N,N-dimethylacetamide are placed in a reaction container, followed by stirring and dissolution. 2.71 g of 3-chloropropionyl chloride is dripped thereto in 15 minutes while cooled with ice. The temperature of the resultant is made back to room temperature and stirred for 180 minutes. Thereafter, 6 ml of triethylamine is dripped in 30 minutes followed by a 14 hour stirring at 60 °C. The reaction solution is poured into 350 ml of water and the insoluble matter is removed. The product is extracted by 300 ml of ethyl acetate and then washed with water three times. The solvent is removed by an evaporator to obtain 7.1 g of a coarse product. The coarse product is dissolved in 125 ml of toluene and 5 g of activated white earth (manufactured by Kanto Chemical Co., Inc.) which has been dried at 130 °C beforehand is added thereto followed by a 30 minute stirring. The activated white earth is removed with filtration and the filtered resultant is condensed with an evaporator. The resultant is column fined using silica gel (60 N, manufactured by Merck Ltd.) with toluene as a developing solvent and thus, 6.51 g of the target substance (non-color oil) of 4-(4'-(di-p-tolylamino)biphenyl-4-yl) butyl acrylate is obtained. The oil is ionized by an atmospheric pressure chemical ionization method. The m/z value measured in the positive mode is 476 and matches the value obtained by adding 1 of proton to the molecular weight calculated by the molecular formula. The IR spectrum is measured and the result thereof is shown in Fig. 14. Synthesis of Illustrated Compound No. 35

25 g of 5-phenyl-1-pentanol is dissolved in 120 ml of acetic acid and 0.5 ml of concentrated sulfuric acid is added to the solution followed by a 120 minute stirring at 65 °C. Thereafter, 17.36 g of iodine and 6.93 g of periodic acid are added thereto to conduct a reaction for 5 hours at 70 °C. The reaction liquid is poured into 400 ml of water and stirred. The supernatant solution is discarded and sodium sulfite aqueous solution is added followed by a 5 hour stirring. The oil layer is washed with water several times and diluted with methylene chloride and dehydrated with magnesium sulfate. The solvent is removed to obtain 48.6 g of the light yellow colored oily target substance. According to a chemical composition analysis by a high speed liquid chromatograph mass spectrometer, the substance is a mixture of 79 % of 5-(4-iodophenyl) pentyl acetate and 21 % of 5-(2-iodophenyl) pentyl acetate.

40 g of the mixture of 5-(4-iodophenyl)pentyl acetate and 5-(2-iodophenyl)pentyl acetate prepared above is dissolved in 105 ml of ethanol and an aqueous solution in which 6.74 g of sodium hydrate dissolved in 76 ml of water is added thereto. Subsequent to a 60 minute stirring at 65 °C, the reaction solution is poured into 400 ml of water and 7.5 ml of concentrated hydrochloric acid is added for neutralization. The oil layer is washed with water several times and diluted with methylene chloride and dehydrated with magnesium sulfate. The solvent is removed to obtain 23.11 g of the light yellow colored oily target substance. According to a chemical composition analysis by a high speed liquid chromatograph mass spectrometer, the substance is a mixture of 79 % of 5-(4-iodophenyl)-1-pentanol and 21 % of 5-(2-iodophenyl)-1-pentanol.

The following recipe is placed in a reaction container and stirred while subject to ultrasonic wave for 20 minutes in argon gas atmosphere.

| | |
|---|---|
| Mixture of 5-(4-iodophenyl)-1-pentanol and 5-(2-iodophenyl)-1-pentanol prepared above | 8.43 g |
| 4-(di-p-tolylamino)phenyl boronic acid | 7.0 g |
| Toluene | 70 ml |
| Ethanol | 35 ml |
| 2M potassium carbonate aqueous solution | 70 ml |

Thereafter, 0.51 g of tetrakis(triphenylphosphine)palladium(0) is added thereto followed by an 8 hour stirring at 70 °C. After cooled down, the resultant is extracted with toluene and washed with water. Subsequent to removal of the solvent, 11 g of a coarse product is obtained. The coarse product is column fined using silica gel and thus 5.94 g of non-color oil substance of 5-(4'-(di-p-tolylamino)biphenyl-4-yl)-1-pentanol is obtained. According to a chemical composition analysis by a high speed liquid chromatograph mass spectrometer, the absorption peak area ratio at a wavelength of 254 nm is 99.6 % and no product derived from 5-(2-iodophenyl)-1-pentanol is contained.

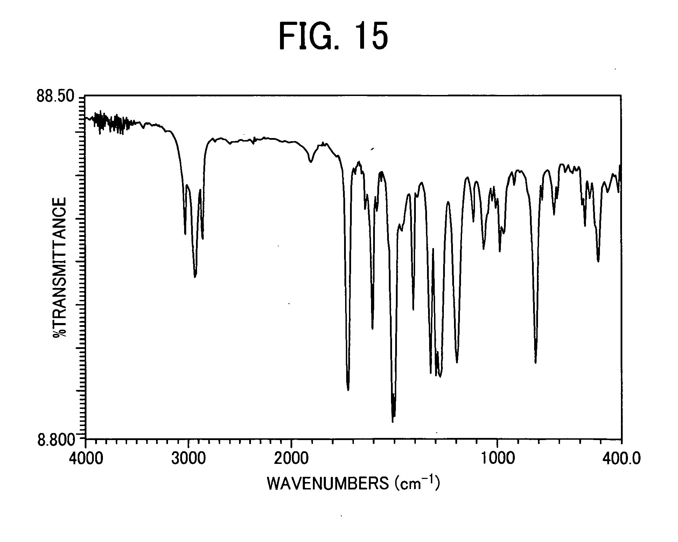

5.9 g of 5-(4'-(di-p-tolylamino)biphenyl-4-yl)-1-propanol and 50 ml of N,N-dimethylacetamide are placed in a reaction container, followed by stirring and dissolution. 2.58 g of 3-chloropropionyl chloride is dripped thereto in 15 minutes while cooled with ice. The temperature of the resultant is made back to room temperature and stirred for 150 minutes. Thereafter, 7.6 ml of triethylamine is dripped in 30 minutes followed by a 4 hour stirring at 60 °C. The reaction solution is poured into 350 ml of water and 0.9 ml of concentrated hydrochloric acid is added in order that pH thereof is 5. The reaction product is extracted by 300 ml of ethyl acetate and then washed with water three times. The solvent is removed by an evaporator and the resultant is dissolved in 125 ml of toluene. 5.5 g of activated white earth (manufactured by Kanto Chemical Co., Inc.) which has been dried at 130 °C beforehand is added thereto followed by a 30 minute stirring. The activated white earth is removed with filtration and the filtered resultant is condensed with an evaporator. The resultant is column fined using silica gel (60 N, manufactured by Merck Ltd.) with toluene as a developing solvent and thus, 6.88 g of the target substance (non-color oil) of 5-(4'-(di-p-tolylamino)biphenyl-4-yl) pentyl acrylate is obtained. The oil is ionized by an atmospheric pressure chemical ionization method. The m/z value measured in the positive mode is 490 and matches the value obtained by adding 1 of proton to the molecular weight calculated by the molecular formula. The IR spectrum is measured and the result thereof is shown in Fig. 15.

Having generally described preferred embodiments of this invention, further understanding can be obtained by reference to certain specific examples which are provided herein for the purpose of illustration only and are not intended to be limiting. In the descriptions in the following examples, the numbers represent weight ratios in parts, unless otherwise specified.

### EXAMPLES

### Example 1

To the surface of an aluminum cylinder having a diameter of 30 mm, the liquid application for an undercoat layer, the liquid application for a charge generating layer and a liquid application for a charge transport layer of the following composition, are sequentially applied and dried to form an undercoat layer having a thickness of 3.5 µm, a charge generating layer having a thickness of 0.2 µm, and a charge transport layer having a thickness of 15 µm. On the charge transport layer, the liquid application for cross linking surface layer is spray coated and naturally dried for 20 minutes followed by irradiation under the following conditions for curing the coated layer. In addition, the coated layer is dried for 20 minutes at 130 °C and an image bearing member of the present invention having a cross linking surface layer of 12 µm is thus obtained.
Metal halide lamp: 160 W/cm
Irradiation distance: 120 mm
Irradiation intensity: 500 mW/cm²
Irradiation time: 120 seconds

### Liquid Application for Undercoating Layer

· Alkyd resin (Beckozole 1307-60-EL, available from Dainippon Ink and Chemicals, Inc.) 6 parts
· Melamine resin (Super- beckamine, available from Dainippon Ink and Chemicals, Inc.) 4 parts
· Titanium oxide 40 parts
· Methylethylketone 50 parts

### Liquid Application for Charge Generation Layer

· Bis-azo pigment represented by the following chemical formula 1 2.5 parts 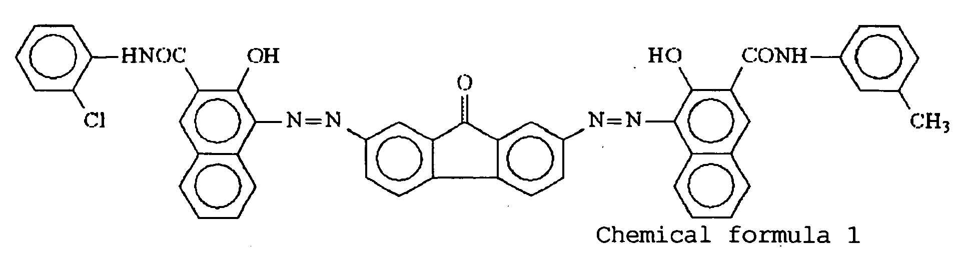
· Polyvinylbutyral (XYHL, manufactured by Union Carbide Corp.) 0.5 parts
· Cyclohexanon 200 parts
· Methylethylketone 80 parts

### Liquid Application for Charge Transport Layer

· Bisphenol Z type polycarbonate (Panlight TS-2050, manufactured by Teijin Chemicals, Ltd.) 10 parts
· Charge transport material represented by the following chemical formula 2 7 parts 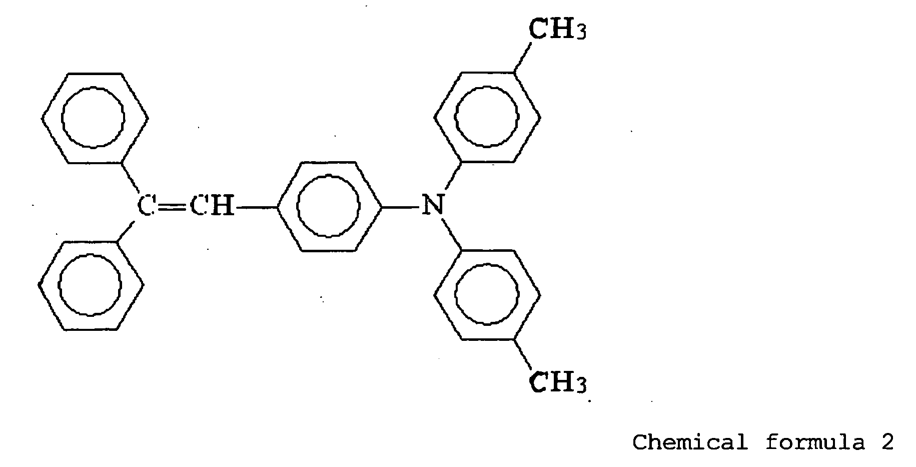
· Tetrahydrofuran 100 parts
· Tetrahydrofuran solution of 1% silicone oil (KF50-100CS, manufactured by Shin-Etsu Chemical Co., Ltd.) 1 part

### Liquid Application for Cross Linking Surface Layer

· Radical reactive compound having the chemical structure of the illustrated compound No. 3 10 parts
· Monomer having three or more radical polymerizable function groups: Trimethylol propane triacrylate (KAYARAD TMPTA, manufactured by Nippon Kayaku Co., Ltd.) 10 parts
· Tetrahydrofuran 100 parts

### Example 2

The image bearing member of Example 2 is manufactured in the same manner as in Example 1 except that the liquid application for cross linking surface layer of Example 1 is changed to the following:

### Liquid application for cross linking surface layer

· Radical reactive compound having the chemical formula of the illustrated compound No. 3 10 parts
· Monomer having three or more radical polymerizable function groups: Trimethylol propane triacrylate (KAYARAD TMPTA, manufactured by Nippon Kayaku Co., Ltd.) 10 parts
· Optical polymerization initiator:
   1-hydroxy-cyclohexyl-phenyl-ketone (IRGACURE 184, manufactured by Chiba Specialty Chemicals K.K.) 1 part
· Tetrahydrofuran 100 parts

### Example 3

The image bearing member of Example 3 is manufactured in the same manner as in Example 1 except that the liquid application for cross linking surface layer of Example 1 is changed to the following:

### Liquid application for Cross Linking Surface Layer

· Radical reactive compound having the chemical formula of the illustrated compound No. 47 10 parts
· Monomer having three or more radical polymerizable function groups: Trimethylol propane triacrylate (KAYARAD TMPTA, manufactured by Nippon Kayaku Co., Ltd.) 10 parts
· Optical polymerization initiator:
   1-hydroxy-cyclohexyl-phenyl-ketone (IRGACURE 184, manufactured by Chiba Specialty Chemicals K.K.) 1 part
· Tetrahydrofuran 100 parts

### Example 4

The image bearing member of Example 4 is manufactured in the same manner as in Example 1 except that the diameter of the aluminum cylinder of Example 1 is changed to 100 mm, and the liquid application for charge generation layer and the liquid application for cross linking surface layer of Example 1 are changed to the following:

### Liquid Application for Charge Generation layer

· Y type titanyl phthalocyanine 2.5 parts
· Polyvinylbutyral (XYHL, manufactured by Union Carbide Corp.) 0.5 parts
· Cyclohexanon 200 parts
· Methylethylketone 80 parts

### Liquid Application for Cross Linking Surface Layer

· Radical reactive compound having the chemical formula of the illustrated compound No. 26 10 parts
· Monomer having three or more radical polymerizable function groups: Trimethylol propane triacrylate (KAYARAD TMPTA, manufactured by Nippon Kayaku Co., Ltd.) 10 parts
· Optical polymerization initiator:
   1-hydroxy-cyclohexyl-phenyl-ketone (IRGACURE 184, manufactured by Chiba Specialty Chemicals K.K.) 1 part
· Tetrahydrofuran 100 parts

### Example 5

The image bearing member of Example 5 is manufactured in the same manner as in Example 4 except that the liquid application for cross linking surface layer of Example 4 is changed to the following:

### Liquid Application for Cross Linking Surface Layer

· Radical reactive compound having the chemical formula of the illustrated compound No. 31 10 parts
· Monomer having three or more radical polymerizable function groups: Trimethylol propane triacrylate (KAYARAD TMPTA, manufactured by Nippon Kayaku Co., Ltd.) 10 parts
· Optical polymerization initiator:
   1-hydroxy-cyclohexyl-phenyl-ketone (IRGACURE 184, manufactured by Chiba Specialty Chemicals K.K.) 1 part
· Tetrahydrofuran 100 parts

### Example 6

The image bearing member of Example 6 is manufactured in the same manner as in Example 4 except that the liquid application for cross linking surface layer of Example 4 is changed to the following:

### Liquid Application for Cross Linking Surface Layer

· Radical reactive compound having the chemical formula of the illustrated compound No. 14 10 parts
· Monomer having three or more radical polymerizable function groups: Trimethylol propane triacrylate (KAYARAD TMPTA, manufactured by Nippon Kayaku Co., Ltd.) 10 parts
· Optical polymerization initiator:
   1-hydroxy-cyclohexyl-phenyl-ketone (IRGACURE 184, manufactured by Chiba Specialty Chemicals K.K.) 1 part
· Tetrahydrofuran 100 parts

### Example 7

The image bearing member of Example 7 is manufactured in the same manner as in Example 1 except that the layer thickness of the cross linking surface layer in Example 1 is changed to 15 µm.

### Example 8

The image bearing member of Example 8 is manufactured in the same manner as in Example 4 except that the layer thickness of the cross linking surface layer in Example 4 is changed to 15 µm.

### Example 9

The image bearing member of Example 9 is manufactured in the same manner as in Example 4 except that the illustrated compound No. 26 of the radical reactive compound is replaced with the illustrated compound No. 1.

### Example 10

The image bearing member of Example 10 is manufactured in the same manner as in Example 4 except that the illustrated compound No. 26 of the radical reactive compound is replaced with the illustrated compound No. 2.

### Example 11

The image bearing member of Example 11 is manufactured in the same manner as in Example 4 except that the illustrated compound No. 26 of the radical reactive compound is replaced with the illustrated compound No. 8.

### Example 12

The image bearing member of Example 12 is manufactured in the same manner as in Example 4 except that the illustrated compound No. 26 of the radical reactive compound is replaced with the illustrated compound No. 9.

### Example 13

The image bearing member of Example 13 is manufactured in the same manner as in Example 4 except that the illustrated compound No. 26 of the radical reactive compound is replaced with the illustrated compound No. 10.

### Example 14

The image bearing member of Example 14 is manufactured in the same manner as in Example 4 except that the illustrated compound No. 26 of the radical reactive compound is replaced with the illustrated compound No. 13.

### Example 15

The image bearing member of Example 15 is manufactured in the same manner as in Example 4 except that the illustrated compound No. 26 of the radical reactive compound is replaced with the illustrated compound No. 16.

### Example 16

The image bearing member of Example 16 is manufactured in the same manner as in Example 4 except that the illustrated compound No. 26 of the radical reactive compound is replaced with the illustrated compound No. 30.

### Example 17

The image bearing member of Example 17 is manufactured in the same manner as in Example 4 except that the illustrated compound No. 26 of the radical reactive compound is replaced with the illustrated compound No. 35.

### Comparative Example 1

The image bearing member of comparative Example 1 is manufactured in the same manner as in Example 1 except that the cross linking surface layer of Example 1 is not provided and the layer thickness of the charge transport layer of Example 1 is changed to 27 µm.

### Comparative Example 2

The image bearing member of comparative Example 2 is manufactured in the same manner as in Example 4 except that the cross linking surface layer of Example 1 is not provided and the layer thickness of the charge transport layer of Example 4 is changed to 27 µm.

### Comparative Example 3

The image bearing member of comparative Example 3 is manufactured in the same manner as in Example 1 except that the radical reactive compound for use in the liquid application for cross linking surface layer of Example 1 is changed to a compound represented by the following chemical formula 3:

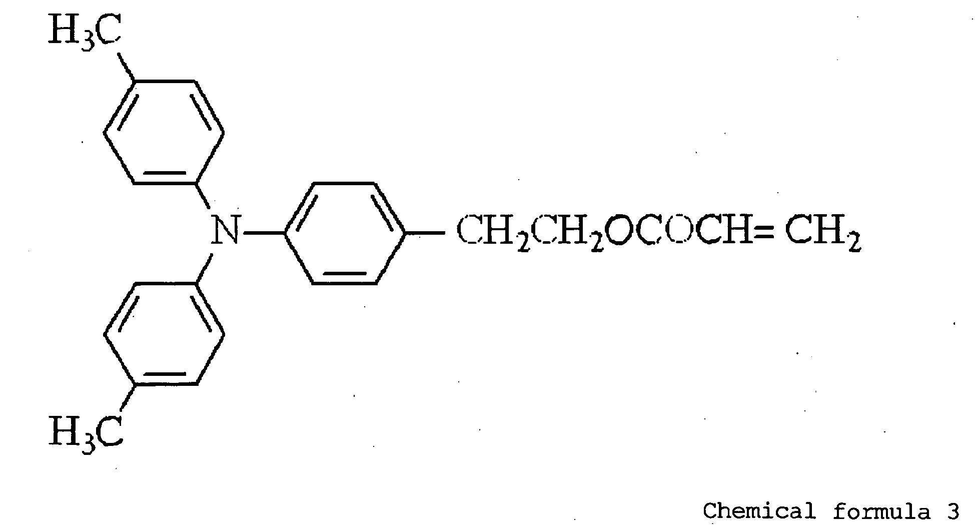

### Comparative Example 4

The image bearing member of comparative Example 4 is manufactured in the same manner as in Example 2 except that the radical reactive compound for use in the liquid application for cross linking surface layer of Example 2 is changed to a compound represented by the following chemical formula 3:

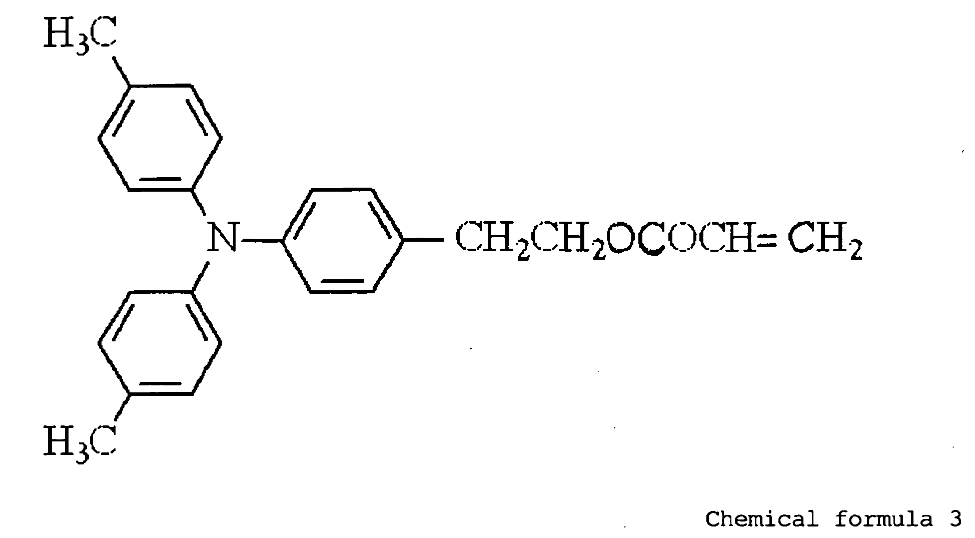

### Comparative Example 5

The image bearing member of comparative Example 5 is manufactured in the same manner as in Example 2 except that the radical reactive compound for use in the liquid application for cross linking surface layer of Example 2 is changed to a compound represented by the following chemical formula 4:

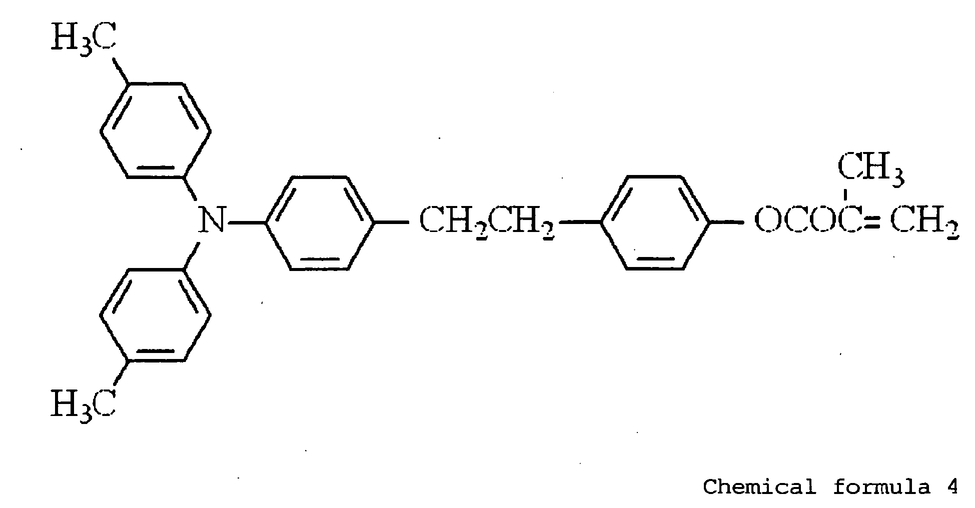

### Comparative Example 6

The image bearing member of comparative Example 6 is manufactured in the same manner as in Example 4 except that the radical reactive compound for use in the liquid application for cross linking surface layer of Example 4 is changed to a compound represented by the following chemical formula 5

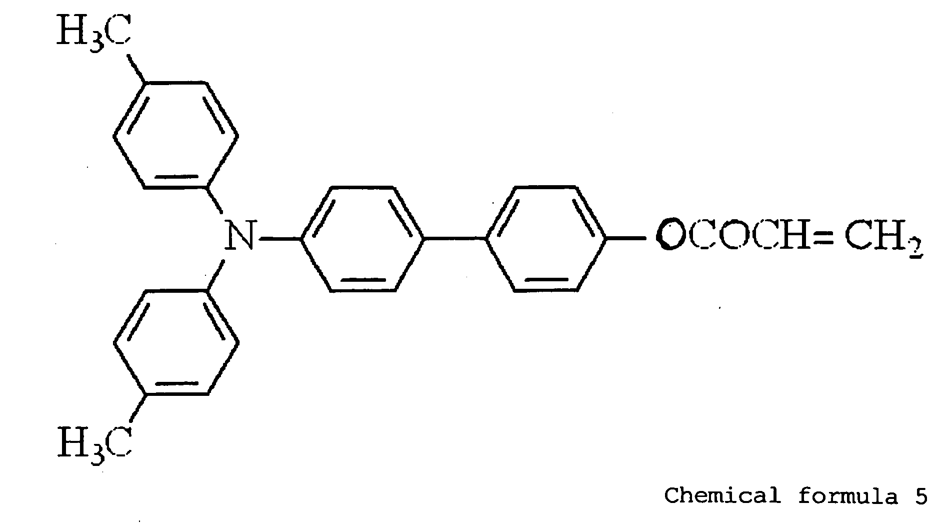

### Comparative Example 7

The image bearing member of comparative Example 7 is manufactured in the same manner as in Example 4 except that the radical reactive compound for use in the liquid application for cross linking surface layer of Example 4 is changed to a compound represented by the following chemical formula 6:

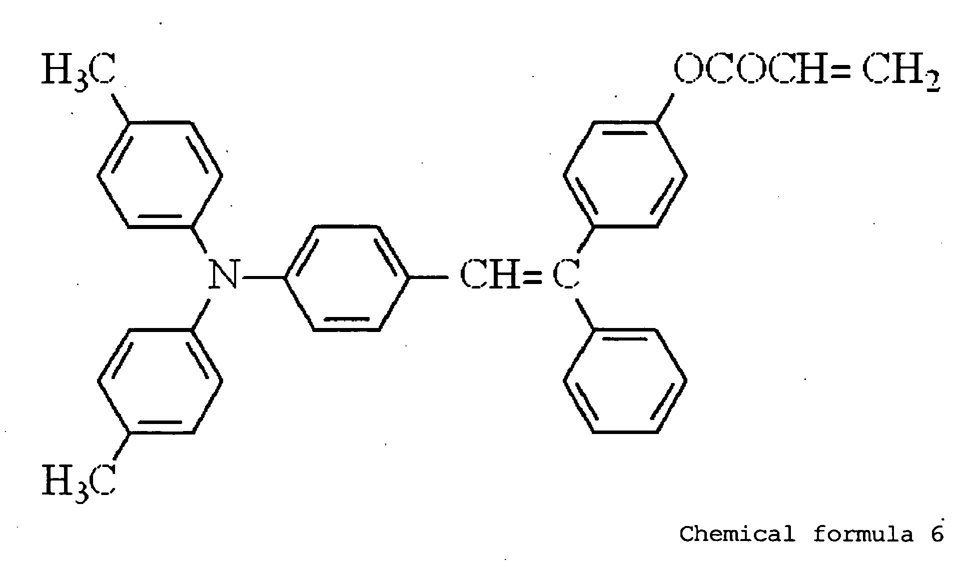

### Comparative Example 8

The image bearing member of comparative Example 8 is manufactured in the same manner as in Example 4 except that the radical reactive compound for use in the liquid application for cross linking surface layer of Example 4 is changed to a compound represented by the following chemical formula 7:

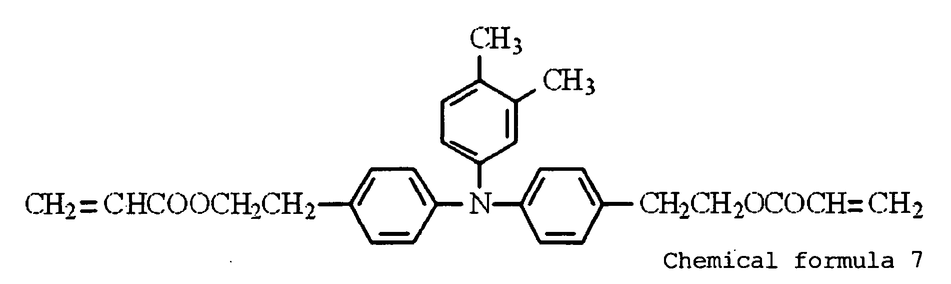

### Comparative Example 9

The image bearing member of comparative Example 9 is manufactured in the same manner as in Example 4 except that the radical reactive compound for use in the liquid application for cross linking surface layer of Example 4 is changed to a compound represented by the following chemical formula 8:

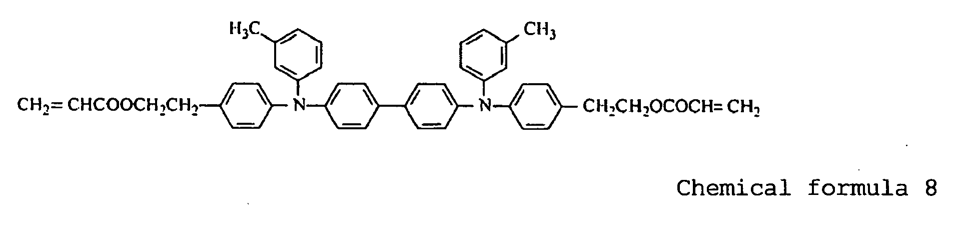

### Comparative Example 10

The image bearing member of comparative Example 10 is manufactured in the same manner as in Example 4 except that the radical reactive compound for use in the liquid application for cross linking surface layer of Example 4 is changed to a compound represented by the following chemical formula 9:

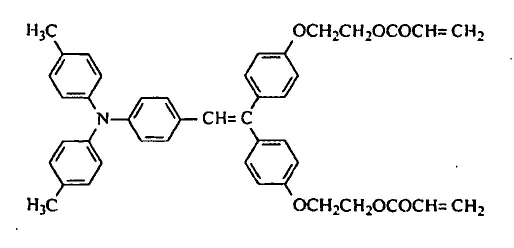

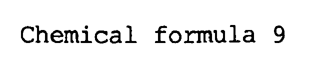

### Comparative Example 11

The image bearing member of comparative Example 11 is manufactured in the same manner as in Example 4 except that the radical reactive compound for use in the liquid application for cross linking surface layer of Example 4 is changed to a compound represented by the following chemical formula 10:

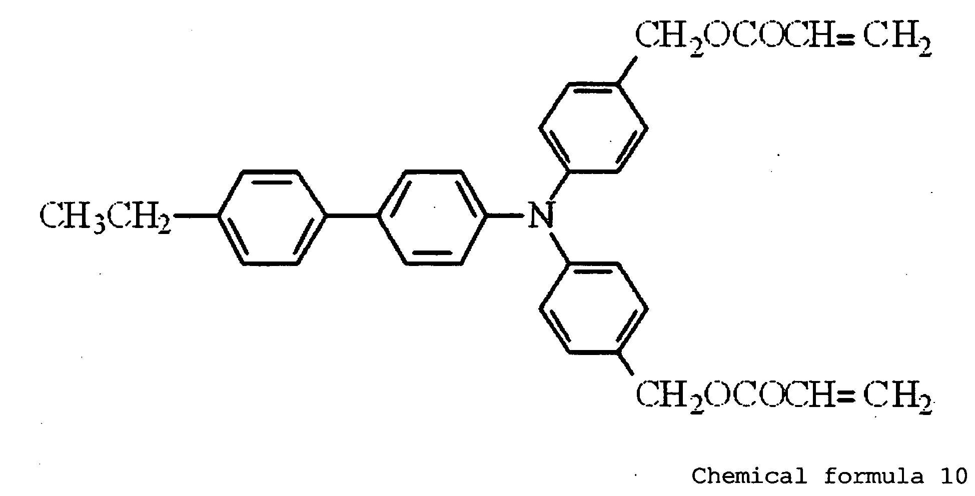

With regard to the image bearing members of Examples 1, 2, 3 and 7 and Comparative Examples 1, 3, 4 and 5 manufactured as described above, a paper run test having a run length of 50,000 A4 sheets at room temperature and normal humidity is performed. The image bearing member is placed in a process cartridge for electrophotography and the process cartridge is installed in a printer (imagio NeoC355, manufactured by Ricoh Co., Ltd.) using semiconductor laser having a wavelength of 655 nm as an image irradiation light source for the photocopying of 50,000 images. The amount of abrasion (the value obtained by subtracting the layer thickness of the image bearing member after photocopying from the layer thickness before photocopying) and the surface voltage at the development portion for a solid image (the values for the initial photocopying and after 50, 000 image formation) are measured. The results are shown in Table 2.

In addition, for the image bearing members of Examples 4, 5, 6 and 8 to 17 and Comparative Examples 2 and 6 to 11 manufactured as described above, a paper run test having a run length of 500,000 A4 sheets at room temperature and normal humidity is performed. The image bearing member is set in a printer (imagio Neo1050Pro, manufactured by Ricoh Co., Ltd.) using a semiconductor laser having a wavelength of 780 nm as an image irradiation light source for the photocopying of 500,000 images. The amount of abrasion (the value obtained by subtracting the layer thickness of the image bearing member after photocopying from the layer thickness before photocopying) and the surface voltage at the development portion for a solid image (the values for the initial photocopying and after 50, 000 image formation) are measured while the process control is off. The results are shown in Tables 2.

**Table 2**

| | Abrasion amount (µµm) | Initial Voltage (-V) | Voltage after copying (-V) |
|---|---|---|---|
| Example 1 | 1.2 | 70 | 65 |
| Example 2 | 0.8 | 70 | 65 |
| Example 3 | 0.8 | 60 | 70 |
| Example 4 | 1.1 | 120 | 130 |
| Example 5 | 1 | 110 | 115 |
| Example 6 | 1.1 | 100 | 105 |
| Example 7 | 0.8 | 105 | 85 |
| Example 8 | 1.1 | 125 | 130 |
| Example 9 | 1.0 | 125 | 130 |
| Example 10 | 1.0 | 120 | 125 |
| Example 11 | 1.1 | 110 | 115 |
| Example 12 | 1.1 | 100 | 105 |
| Example 13 | 1.1 | 110 | 115 |
| Example 14 | 1.1 | 105 | 110 |
| Example 15 | 1.1 | 100 | 100 |
| Example 16 | 1.0 | 105 | 110 |
| Example 17 | 1.0 | 100 | 110 |
| Comparative Example 1 | 14.5 | 20 | 50 |
| Comparative Example 2 | 13.6 | 50 | 110 |
| Comparative Example 3 | 0.9 | 220 | 265 |
| Comparative Example 4 | 1 | 220 | 265 |
| Comparative Example 5 | 1.5 | 215 | 260 |
| Comparative Example 6 | 1.1 | 200 | 210 |
| Comparative Example 7 | 1.8 | 180 | 220 |
| Comparative Example 8 | 0.8 | 235 | 260 |
| Comparative Example 9 | 0.9 | 150 | 210 |
| Comparative Example 10 | 0.8 | 150 | 220 |
| Comparative Example 11 | 0.8 | 240 | 180 |

The image bearing members of Examples of the present invention have by far better anti-abrasion property than a typical organic image bearing member having no protective layer. In comparison with a typical organic image bearing member having a charge transport curing protective layer, the image bearing members of Examples are at the best level with regard to the anti-abrasion property, have an excellent voltage decay characteristic and can maintain the voltage decay characteristic while variously fatigued over the operation. Furthermore, the image bearing members of Examples have the characteristics mentioned above even though the image bearing members have a relatively thick protective layer in comparison with a typical protective layer. Due to these characteristics, the image bearing member of the present invention has an extremely long life.

With regard to the anti-abrasion property, several image bearing members of Comparative Examples are good, but the surface voltage at the development portion is already high at the beginning for a solid image for all the image bearing members of Comparative Examples. Therefore, the voltage difference between the irradiated portion and the non-irradiated portion is small so that the image density tends to fluctuate. By contrast, in Examples of the present invention, the image bearing members are good at charge transport and the surface voltage of the development portion is small from the initial and the variation thereof is small even after the image bearing members are fatigued. Consequently, the image bearing members of the present invention can produce quality images stably for an extended period of time.

When Example 1 is compared with Examples 2 and 3, it is found that the anti-abrasion property is improved due to the initiator. Therefore, it is possible to provide an image bearing member having excellent anti-abrasion property when an initiator is used in combination.

Therefore, by using the image bearing member of the present invention, it is possible to provide a reliable high performance image forming apparatus and process cartridge which can produce quality images for an extended period of time.

To check the ease of use of the liquid application for the cross linking surface layer for use in manufacturing the image bearing member of Examples 2 to 6 and 9 to 17, the applied layer before irradiation of ultraviolet is left for one day and whether the applied layer is kept uniform is observed by naked eyes. In addition, the applied layer is irradiated with ultraviolet for cross-lniking and the state thereof is observed by naked eyes. As a result, each sample of the applied layer before irradiation of ultraviolet observed after a short while is smooth and uniform. However, for each sample of the applied layer before irradiation of ultraviolet left for one day, turbidity is observed in the liquid application for the cross linking surface layer for use in Example 9 due to phase separation and remains after cross-linked. Furthermore, turbidity is slightly observed in the liquid applications for the cross linking surface layer for use in Examples 2 and 16 and remains after cross-linked. When the physical propertyies of the radical reactive compounds for use in the samples in which turbidity is observed are compared with those for use in the samples in which turbidity is not observed, the occurrence of turbidity depends on whether the radical reactive compounds are obtained as crystal, or amorphous or oily substance as in the synthesis results. That is, the radical reactive compounds obtained as crystal are easily crystallized so that crystallization occurs when the compounds are left for a long time, which leads to phase separation. On the other hand, the radical reactive compounds obtained as amorphous or oily substance are hardly crystallized, which is considered to be a reason for avoiding phase separation. The degree of ease of crystallization relates to the length of the alkylene group represented by X and the symmetry property of the phenyl groups connected to the nitrogen atom in Chemical formula B. That is, when X in Chemical formula B is ethylene group, which has 2 carbon atoms, and the phenyl group having R₁ to R₅ and the phenyl group having R₁' to R₅' are different from each other (i.e., assymeetric), crystallization hardly occurs. In addition, when X in Chemical formula B is a straight chain alkylene group having 3 to 5 carbon atoms, crystallization hardly occurs regardless of the combination of the phenyl group having R₁ to R₅ and the phenyl group having R₁' to R₅'.

It is not necessarily necessary to use a structure which is hardly crystallized since the application and cross-linking conditions can be arbitrarily controlled. However, it is preferred to use a structure which is hardly crystallized because such a structure has a wide applicability to a wide range of changes and variances in application environments, solvent species, monomer compositions, etc. so that an image bearing member having a stable and uniform crosslinked layer can be manufactured in a wide latitude of design flexibility.

## Claims

1. An image bearing member comprising:
an electroconductive substrate;
a photosensitive layer located overlying the electroconductive substrate; and
a crosslinked surface layer comprising a charge transport curing compound and located overlying the photosensitive layer,
wherein the charge transport curing compound comprises a material comprising a structure unit represented by the following Chemical formula A: 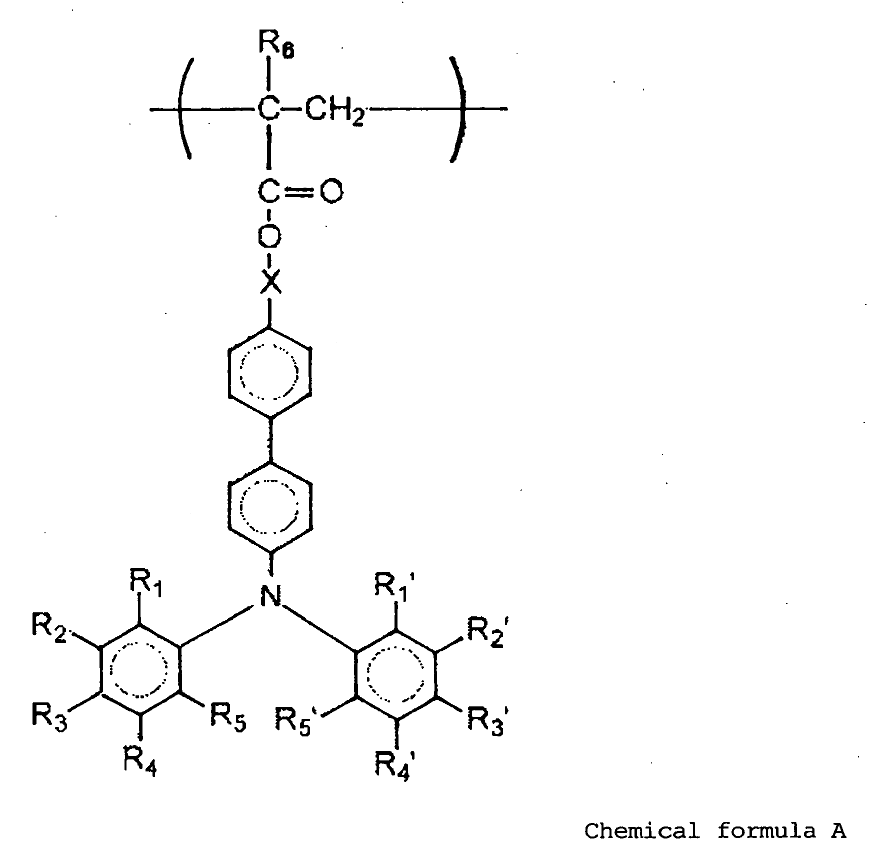 where R₁ to R₅ and R₁' to R₅' independently represent a hydrogen atom, an alkyl group having 1 to 4 carbon atoms or an alkoxy group having 1 to 4 carbon atoms, R₆ represents a hydrogen atom or a methyl group and X represents an alkylene group having a straight chain or a branched chain having 2 to 5 carbon atoms.

2. The image bearing member according to Claim 1, wherein X is an ethylene group and a phenyl group having R₁ to R₅ groups is different from a phenyl group having R₁' to R₅' groups.

3. The image bearing member according to Claim 1, wherein X is a straight chain alkylene group having 3 to 5 carbon atoms.

4. The image bearing member according to any one of Claims 1 to 3, wherein the charge transport curing compound is formed by applying a liquid application comprising the compound represented by Chemical formula A and a monomer having at least 3 polymerizable function groups to a surface of the photosensitive layer followed by curing.

5. The image bearing member according to Claim 4, wherein the liquid application further comprises a polymerization initiator.

6. The image bearing member according to any one of Claims 1 to 5, wherein the crosslinked surface layer is formed on the photosensitive layer and the photosensitive layer is uncured.

7. The image bearing member according to any one of Claims 1 to 6, wherein the charge transport curing compound is formed by a device using optical energy.

8. The image bearing member according to any one of Claims 1 to 7, wherein the photosensitive layer has a structure in which a charge generation layer and a charge transport layer are sequentially accumulated on the electroconductive substrate.

9. A method of manufacturing an image bearing member comprising:
coating a liquid application comprising a radical reactive compound represented by the following Chemical formula B to a surface of the photosensitive layer of the image bearing member of Claim 1; and
curing the liquid application to form the charge transport curing compound; 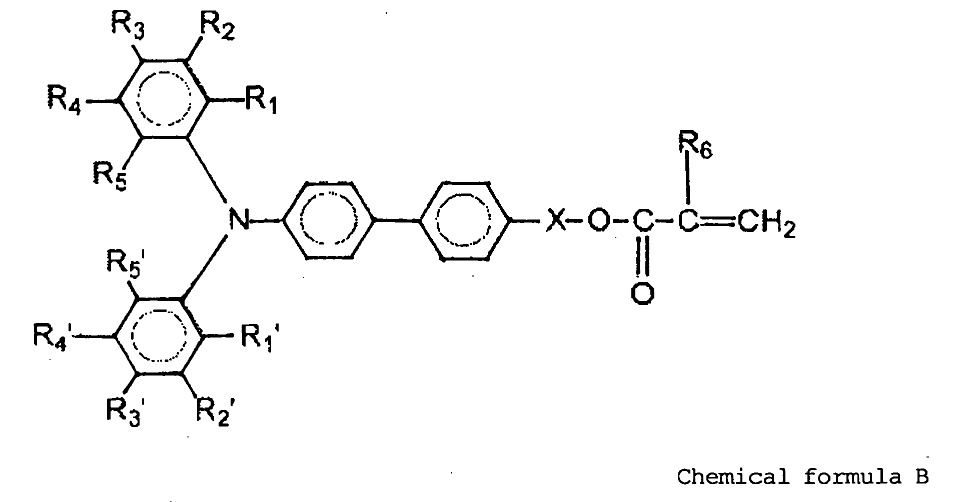 where R₁ to R₅ and R₁' to R₅' independently represent a hydrogen atom, an alkyl group having 1 to 4 carbon atoms or an alkoxy group having 1 to 4 carbon atoms, R₆ represents a hydrogen atom or a methyl group and X represents an alkylene group having 2 to 5 carbon atoms with a straight chain or a branched chain.

10. The method of manufacturing an image bearing member according to Claim 9, wherein X in the chemical formula B is an ethylene group and a phenyl group having R₁ to R₅ groups is different from a phenyl group having R₁' to R₅' groups.

11. The method of manufacturing an image bearing member according to Claim 9, wherein X in the chemical formula B is a straight chain alkylene group having 3 to 5 carbon atoms.

12. An image formation method comprising:
charging the image bearing member of Claim 1;
irradiating the image bearing member to form a latent electrostatic image on the image bearing member;
developing the latent electrostatic image with a developing agent to form a developed image; and
transferring the developed image to a recording medium.

13. An image forming apparatus comprising:
the image bearing member of Claim 1;
a charging device configured to charge the image bearing member;
an irradiation device configured to irradiate the image bearing member with light to form a latent electrostatic image on the image bearing member;
a development device configured to develop the latent electrostatic image with a developing agent;
a transfer device configured to transfer the developed image to a recording medium.

14. A process cartridge comprising:
the image bearing member of Claim 1; and
at least one device selected from the group consisting of a charging device, an irradiation device, a development device, a transfer device, a cleaning device and a discharging device,
wherein the image bearing member and the at least one device are integrally structured and the process cartridge is detachably attachable to an image forming apparatus.

15. A radical reactive compound represented by chemical formula B: 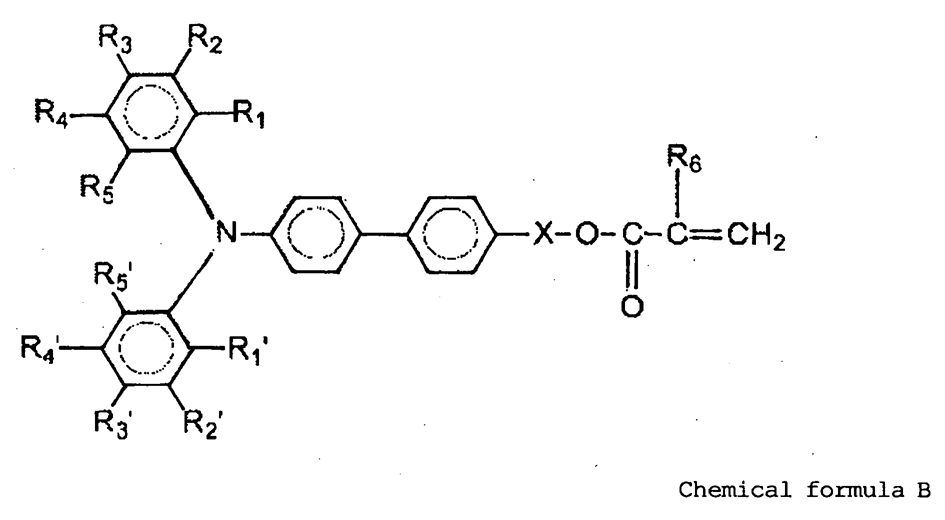 where R₁ to R₅ and R₁' to R₅' independently represent a hydrogen atom, an alkyl group having 1 to 4 carbon atoms or an alkoxy group having 1 to 4 carbon atoms, R₆ represents a hydrogen atom or a methyl group, X represents an alkylene group having 2 to 5 carbon atoms with a straight chain or a branched chain and when a number of carbon atoms in X is two, a phenyl group having R₁ to R₅ groups is different from a phenyl group having R₁' to R₅' groups.

## Patentansprüche

1. Bildträgerelement umfassend:
ein elektrisch leitfähiges Substrat;
eine über dem elektrisch leitfähigen Substrat angeordnete lichtempfindliche Schicht; und
eine vernetzte Oberflächenschicht, umfassend eine Ladungstransport-Härtungsverbindung und angeordnet über der lichtempfindlichen Schicht,
wobei die Ladungstransport-Härtungsverbindung eine Struktureinheit umfasst, die durch die folgende chemische Formel A dargestellt wird: 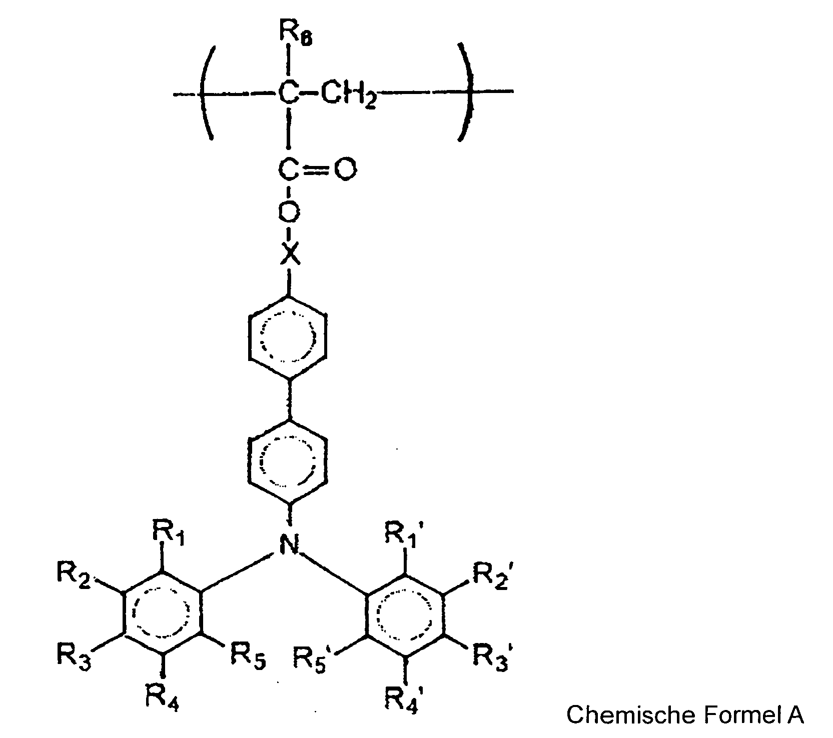 wobei R₁ bis R₅ und R₁' bis R₅' unabhängig voneinander ein Wasserstoffatom, eine Alkylgruppe mit 1 bis 4 Kohlenstoffatomen oder eine Alkoxygruppe mit 1 bis 4 Kohlenstoffatomen darstellen, R₆ ein Wasserstoffatom oder eine Methylgruppe darstellt und X eine Alkylengruppe mit einer geraden Kette oder einer verzweigten Kette mit 2 bis 5 Kohlenstoffatomen darstellt.

2. Bildträgerelement gemäß Anspruch 1, wobei X eine Ethylengruppe ist und eine Phenylgruppe mit den Gruppen R₁ bis R₅ von einer Phenylgruppe mit den Gruppen R₁' bis R₅' verschieden ist.

3. Bildträgerelement gemäß Anspruch 1, wobei X eine geradkettige Alkylengruppe mit 3 bis 5 Kohlenstoffatomen ist.

4. Bildträgerelement gemäß irgendeinem der Ansprüche 1 bis 3, wobei die Ladungstransport-Härtungsverbindung erzeugt wird, indem eine Liquid-Applikation, umfassend die durch die chemische Formel A dargestellte Verbindung und ein Monomer mit mindestens 3 polymerisierbaren Funktionsgruppen, auf die Oberfläche der lichtempfindlichen Schicht aufgebracht wird, gefolgt von Härtung.

5. Bildträgerelement gemäß Anspruch 4, wobei die Liquid-Applikation ferner einen Polymerisationsinitiator umfasst.

6. Bildträgerelement gemäß irgendeinem der Ansprüche 1 bis 5, wobei die vernetzte Oberflächenschicht auf der lichtempfindlichen Schicht erzeugt wird und die lichtempfindliche Schicht ungehärtet ist.

7. Bildträgerelement gemäß irgendeinem der Ansprüche 1 bis 6, wobei die Ladungstransport-Härtungsverbindung durch eine Vorrichtung erzeugt wird, die optische Energie verwendet.

8. Bildträgerelement gemäß irgendeinem der Ansprüche 1 bis 7, wobei die lichtempfindliche Schicht eine Struktur aufweist, in welcher eine Ladungserzeugungsschicht und eine Ladungstransportschicht nacheinander auf dem elektrisch leitfähigen Substrat angesammelt sind.

9. Verfahren zur Herstellung eines Bildträgerelementes umfassend:
Beschichten einer Liquid-Applikation umfassend eine durch die folgende chemische Formel B dargestellte radikal-reaktive Verbindung auf eine Oberfläche der lichtempfindlichen Schicht des Bildträgerelementes gemäß Anspruch 1; und
Härten der Liquid-Applikation, um die Ladungstransport-Härtungsverbindung zu erzeugen; 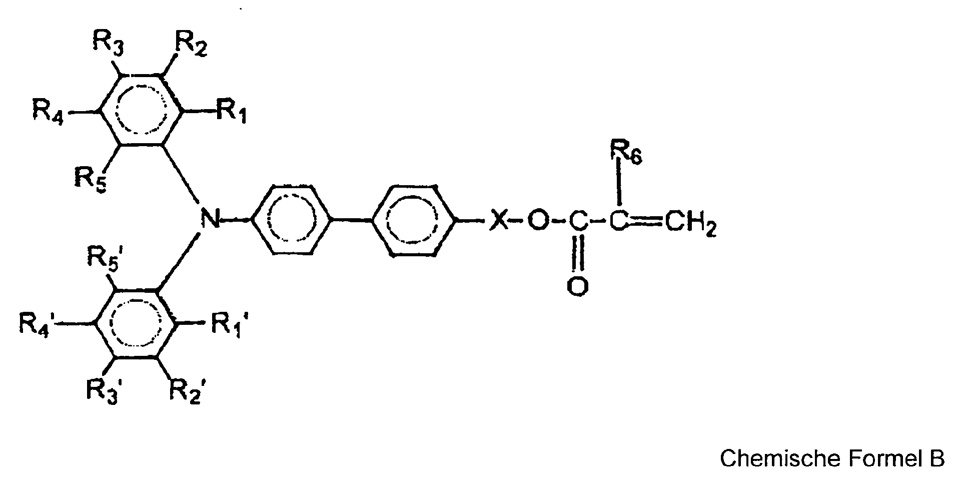 wobei R₁ bis R₅ und R₁' bis R₅' unabhängig voneinander ein Wasserstoffatom, eine Alkylgruppe mit 1 bis 4 Kohlenstoffatomen oder eine Alkoxygruppe mit 1 bis 4 Kohlenstoffatomen darstellen, R₆ ein Wasserstoffatom oder eine Methylgruppe darstellt und X eine Alkylengruppe mit 2 bis 5 Kohlenstoffatomen mit einer geraden Kette oder einer verzweigten Kette darstellt.

10. Verfahren zur Herstellung eines Bildträgerelementes gemäß Anspruch 9, wobei X in der chemischen Formel B eine Ethylengruppe ist und eine Phenylgruppe mit den Gruppen R₁ bis R₅ von einer Phenylgruppe mit den Gruppen R₁' bis R₅' verschieden ist.

11. Verfahren zur Herstellung eines Bildträgerelementes gemäß Anspruch 9, wobei X in der chemischen Formel B eine geradkettige Alkylengruppe mit 3 bis 5 Kohlenstoffatomen ist.

12. Bilderzeugungsverfahren umfassend:
Aufladen des Bildträgerelementes gemäß Anspruch 1;
Bestrahlen des Bildträgerelementes, um auf dem Bildträgerelement ein latentes elektrostatisches Bild zu erzeugen;
Entwickeln des latenten elektrostatischen Bildes mit einem Entwicklungsmittel, um ein entwickeltes Bild zu erzeugen; und
Übertragen des entwickelten Bildes auf ein Aufzeichnungsmedium.

13. Bilderzeugungsvorrichtung umfassend:
das Bildträgerelement gemäß Anspruch 1;
eine Aufladungsvorrichtung, konfiguriert zum Aufladen des Bildträgerelementes;
eine Bestrahlungsvorrichtung, konfiguriert zum Bestrahlen des Bildträgerelementes mit Licht, um auf dem Bildträgerelement ein latentes elektrostatisches Bild zu erzeugen;
eine Entwicklungsvorrichtung, konfiguriert das latente elektrostatische Bild mit einem Entwicklungsmittel zu entwickeln;
eine Übertragungsvorrichtung, konfiguriert das entwickelte Bild auf ein Aufzeichnungsmedium zu übertragen.

14. Prozesskartusche umfassend:
das Bildträgerelement gemäß Anspruch 1; und
mindestens eine Vorrichtung ausgewählt aus der Gruppe bestehend aus einer Aufladungsvorrichtung, einer Bestrahlungsvorrichtung, einer Entwicklungsvorrichtung, einer Übertragungsvorrichtung und einer Entladungsvorrichtung,
wobei das Bildträgerelement und die mindestens eine Vorrichtung integral strukturiert sind und die Prozesskartusche abnehmbar an einer Bilderzeugungsvorrichtung anbringbar ist.

15. Radikal-reaktive Verbindung dargestellt durch die chemische Formel B: 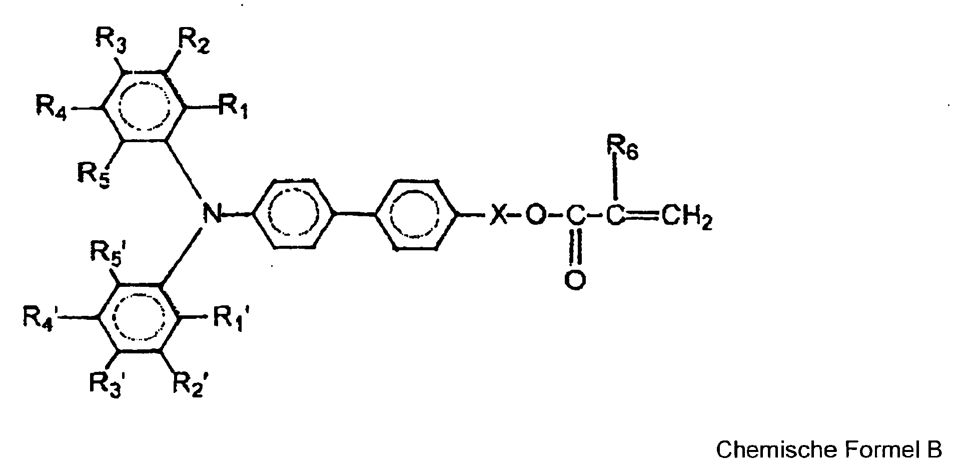 wobei R₁ bis R₅ und R₁' bis R₅' unabhängig voneinander ein Wasserstoffatom, eine Alkylgruppe mit 1 bis 4 Kohlenstoffatomen oder eine Alkoxygruppe mit 1 bis 4 Kohlenstoffatomen darstellen, R₆ ein Wasserstoffatom oder eine Methylgruppe darstellt und X eine Alkylengruppe mit 2 bis 5 Kohlenstoffatomen mit einer geraden Kette oder einer verzweigten Kette darstellt und, wenn die Anzahl der Kohlenstoffatome in X zwei ist, eine Phenylgruppe mit den Gruppen R₁ bis R₅ von einer Phenylgruppe mit den Gruppen R₁' bis R₅' verschieden ist.

## Revendications

1. Elément supportant une image comprenant :
un substrat électroconducteur ;
une couche photosensible disposée sur le substrat électroconducteur ; et
une couche de surface réticulée comprenant le composé durcissant de transport de charge et disposée sur la couche photosensible,
dans lequel le composé durcissant de transport de charge comprend un matériau comprenant une unité de structure représentée par la formule chimique A suivante : 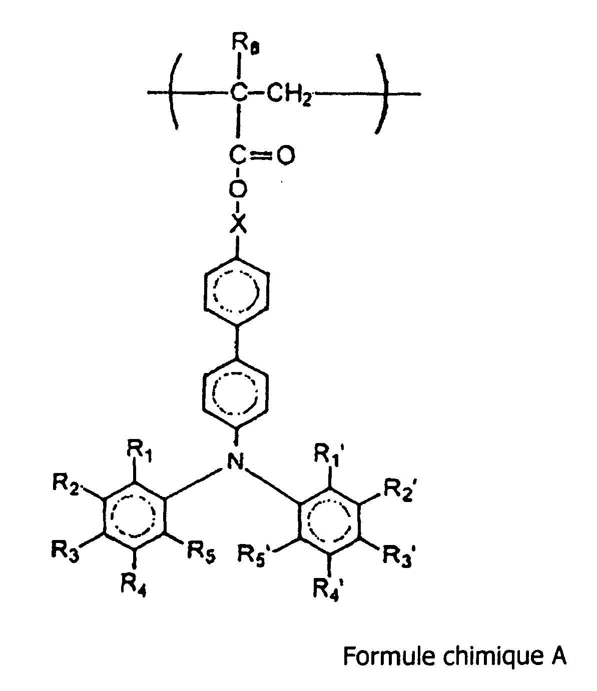 dans laquelle R₁ à R₅ et R'₁ à R'₅ représentent indépendamment un atome d'hydrogène, un groupe alkyle ayant de 1 à 4 atomes de carbone ou un groupe alcoxy ayant de 1 à 4 atomes de carbone, R₆ représente un atome d'hydrogène ou un groupe méthyle et X représente un groupe alkylène ayant une chaîne linéaire ou une chaîne ramifiée de 2 à 5 atomes de carbone.

2. Elément supportant une image selon la revendication 1, dans lequel X est un groupe éthylène et un groupe phényle présentant les groupes R₁ à R₅ est différent d'un groupe phényle présentant les groupes R₁' à R₅'.

3. Elément supportant une image selon la revendication 1, dans lequel X est un groupe alkylène à chaîne linéaire ayant de 3 à 5 atomes de carbone.

4. Elément supportant une image selon l'une quelconque des revendications 1 à 3, dans lequel le composé durcissant de transport de charge est formé par application d'une application liquide comprenant le composé représenté par la formule chimique A et un monomère présentant au moins trois groupes fonctionnels polymérisables sur une surface de la couche photosensible suivie par un durcissement.

5. Elément supportant une image selon la revendication 4, dans lequel l'application liquide comprend de plus un initiateur de polymérisation.

6. Elément supportant une image selon l'une quelconque des revendications 1 à 5, dans lequel la couche de surface réticulée est formée sur la couche photosensible et la couche photosensible est non durcie.

7. Elément supportant une image selon l'une quelconque des revendications 1 à 6, dans lequel le composé durcissant de transport de charge est formé par un dispositif utilisant une énergie optique.

8. Elément supportant une image selon l'une quelconque des revendications 1 à 7, dans lequel la couche photosensible présente une structure dans laquelle une couche de génération de charge et une couche de transport de charge sont successivement accumulées sur le substrat électroconducteur.

9. Procédé de fabrication d'un élément supportant une image comprenant :
le dépôt d'une application liquide comprenant un composé réactif radicalaire représenté par la formule chimique B suivante sur une surface de la couche photosensible de l'élément supportant une image selon la revendication 1 ; et
le durcissement de l'application liquide pour former le composé
durcissant de transport de charge ; 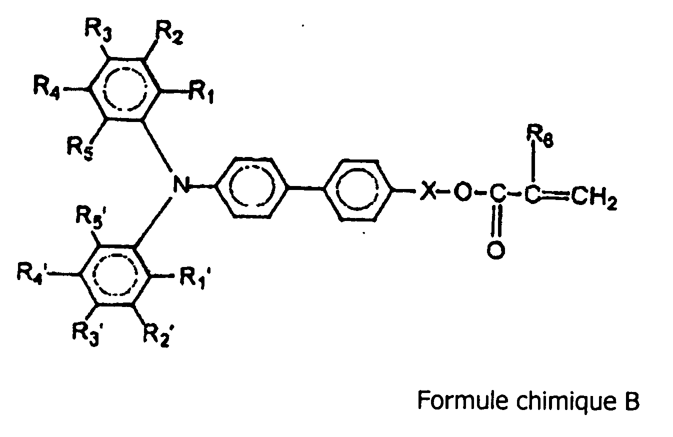 dans laquelle R₁ à R₅ et R'₁ à R'₅ représentent indépendamment un atome d'hydrogène, un groupe alkyle ayant de 1 à 4 atomes de carbone ou un groupe alcoxy ayant de 1 à 4 atomes de carbone, R₆ représente un atome d'hydrogène ou un groupe méthyle et X représente un groupe alkylène ayant de 2 à 5 atomes de carbone avec une chaîne linéaire ou une chaîne ramifiée.

10. Procédé de fabrication d'un élément supportant une image selon la revendication 9, dans lequel X dans la formule chimique B est un groupe éthylène et un groupe phényle présentant les groupes R₁ à R₅ est différent d'un groupe phényle présentant les groupes R₁' à R₅'.

11. Procédé de fabrication d'un élément supportant une image selon la revendication 9, dans lequel X dans la formule chimique B est un groupe alkylène à chaîne linéaire ayant de 3 à 5 atomes de carbone.

12. Procédé de formation d'une image comprenant :
le chargement de l'élément supportant une image selon la revendication 1 ;
l'irradiation de l'élément supportant une image pour former une image électrostatique latente sur l'élément supportant une image ;
le développement de l'image électrostatique latente avec un agent de développement pour former une image développée ; et
le transfert de l'image développée sur un support d'enregistrement.

13. Appareil formant une image comprenant :
l'élément supportant une image selon la revendication 1 ;
un dispositif de chargement configuré pour charger l'élément supportant une image ;
un dispositif d'irradiation configuré pour irradier l'élément supportant une image avec de la lumière afin de former une image électrostatique latente sur l'élément supportant une image ;
un dispositif de développement configuré pour développer l'image électrostatique latente avec un agent de développement ;
un dispositif de transfert configuré pour transférer l'image développée sur un support d'enregistrement.

14. Cartouche de procédé comprenant :
l'élément supportant une image selon la revendication 1 ; et
au moins un dispositif choisi dans le groupe constitué d'un dispositif de chargement, d'un dispositif d'irradiation, d'un dispositif de développement, d'un dispositif de transfert, d'un dispositif de nettoyage et d'un dispositif de déchargement,
dans laquelle l'élément supportant une image et le au moins un dispositif sont structurés en une seule pièce et la cartouche de procédé peut être fixée de manière détachable à un appareil formant une image.

15. Composé réactif radicalaire représenté par la formule chimique B : 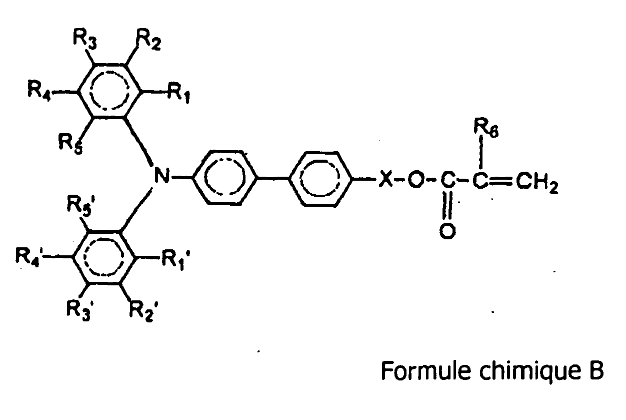 dans laquelle R₁ à R₅ et R'₁ à R'₅ représentent indépendamment un atome d'hydrogène, un groupe alkyle ayant de 1 à 4 atomes de carbone ou un groupe alcoxy ayant de 1 à 4 atomes de carbone, R₆ représente un atome d'hydrogène ou un groupe méthyle, X représente un groupe alkylène ayant de 2 à 5 atomes de carbone avec une chaîne linéaire ou une chaîne ramifiée et lorsque le nombre d'atomes de carbone dans X est égal à 2, un groupe phényle présentant les groupes R₁ à R₅ est différent d'un groupe phényle présentant les groupes R₁' à R₅'.
